# EUROPEAN PATENT APPLICATION

(11) **EP 4 803 885 A1**
(43) Date of publication of application: **09.09.2026**
(21) Application number: 24884887.1
(22) Date of filing: 31.10.2024
(51) Int. Cl.: G01N 21/64, A61K 49/00

(54) **PROBE AND USE THEREOF IN IMAGING AND THERAPY**

(30) Priority: 31.10.2023 CN 202311432730
(71) Applicant: Xiang an Biomedicine Laboratory, Fujian 361104 (CN); Xiamen University, Xiamen, Fujian 361005 (CN)
(72) Inventor: TIAN, Rui, Xiamen, Fujian 361005 (CN); CHEN, Yiqing, Xiamen, Fujian 361005 (CN); KE, Chaomin, Xiamen, Fujian 361005 (CN); YANG, Minglei, Xiamen, Fujian 361005 (CN)
(74) Representative: Zanoli, Enrico
(86) International application number: PCT/CN2024/128835
(87) International publication number: WO 2025/092887

(57) **Abstract**

The present invention relates to the technical field of imaging and therapeutic probes, and specifically to a probe and a use thereof in imaging and therapy. The present invention specifically provides a complex, comprising: 1) a protein shell, wherein the protein shell is a third structural domain of albumin or a variant thereof, or a third structural domain a-subunit of albumin or a variant thereof; 2) a small molecule binding to the protein shell; and 3) a functionalized fragment binding to the protein shell, wherein the functionalized fragment is a molecule capable of being applied to diagnosis, prevention or treatment of kidney-bladder clearance pathway diseases. Also provided is a use of the complex.

## Description

### Cross-Reference to Related Applications

The present application is based on and claims priority to the Chinese application with application number CN 202311432730.4 (filed on October 31, 2023). The disclosure of the Chinese application is incorporated into the present application by reference in its entirety.

### Technical Field

The present application relates to the technical field of probes, and in particular to a renal-metabolism-type probe based on an albumin variant and uses thereof.

### Background Art

Bladder cancer is one of the most common cancers worldwide. According to epidemiological statistics, there were approximately 550,000 new cases in 2018. Among urinary system diseases, its incidence ranks second only to prostate cancer. Bladder cancer is mainly caused by proliferation of urothelial cells. Approximately 75% of patients are diagnosed with non-muscle-invasive bladder cancer (NMIBC). At present, the primary treatment for NMIBC is transurethral resection of bladder tumor under cystoscopy, typically followed by intravesical instillation of first-line drugs such as Bacillus Calmette-Guérin (BCG) or mitomycin C to reduce recurrence. However, approximately 60% of patients still experience recurrence, and about 25% of patients show disease progression. Cystoscopic examination is invasive and has limitations, which may lead to missed diagnoses. Although regulatory agencies have recently approved the anti-PD-1 (programmed cell death protein 1) therapeutic agent pembrolizumab for bladder cancer treatment, its efficacy remains limited. Apart from radical cystectomy, few effective salvage treatment options are available. Therefore, there is an urgent need to develop novel therapeutic strategies for high-risk NMIBC.

Imaging methods can provide early diagnosis of bladder cancer as well as non-invasive and precise postoperative monitoring. In addition, photosensitizers can be used for photodynamic therapy under image guidance. Furthermore, radionuclide labeling can enable early imaging (e.g., ⁶⁴Cu, ¹⁸F) and therapy (e.g., therapeutic radionuclides such as ¹⁷⁷Lu).

Near-infrared fluorescence imaging has been widely used in tumor diagnosis and treatment due to its good safety and high tissue penetration. Fluorescent agents used in near-infrared imaging include inorganic nanomaterials (such as carbon nanotubes, quantum dots, and rare-earth nanoparticles) and hydrophobic small-molecule organic dyes. Most inorganic nanomaterials with high quantum yield contain heavy metal components. These materials have poor biocompatibility and may cause long-term toxicity. Relatively ideal alternatives are small-molecule organic dyes with higher biosafety. However, organic molecules with near-infrared emission usually have large conjugated structures. They are easily taken up by the reticuloendothelial system in vivo, and are mainly metabolized through the hepatobiliary and intestinal routes. It is difficult for them to be excreted via the urinary system. This is exemplified by indocyanine green (ICG), a dye approved for clinical use by the U.S. Food and Drug Administration. In addition, although cyanine/polymethine dyes commonly used in organic materials have advantages such as good biocompatibility and near-infrared tail emission, their free dye forms often exhibit low quantum yield and poor pharmacokinetic properties, posing challenges for clinical translation.

### Summary of the Invention

The present invention provides a complex formed by covalent binding of a protein shell and a cyanine dye molecule. In the complex, the cyanine dye molecule serves as a core chromophore, and the protein shell is covalently bound to the exterior thereof. The protein shell can be functionalized through genetic engineering or chemical conjugation. The complex has a relatively small size, and thus can be used for the treatment of diseases in major organs and tissues of the renal-bladder metabolic pathway. Compared with near-infrared fluorescent probes currently used in clinical practice, the complex exhibits improved photostability, higher photothermal conversion efficiency, higher quantum yield, and a significantly extended emission wavelength, rendering it suitable for deep tissue imaging and photodynamic therapy. In particular, the complex provides an effective therapeutic option for high-risk NMIBC patients who are unresponsive to BCG.

Accordingly, the present application provides the following invention:
In a first aspect, the present application provides a complex. The complex comprises: 1) a protein shell, wherein the protein shell is a third domain (domain III, DIII) of albumin or a variant thereof, or the a subunit of the third domain (DIIIa subunit) of albumin or a variant thereof; 2) a small molecule binding to the protein shell; and 3) a functionalized moiety linked to the protein shell, wherein the functionalized moiety is a molecule that can be used for diagnosis, prevention, or treatment of a disease associated with a renal-bladder clearance pathway.

In the present invention, the albumin may be mammalian serum albumin. In some embodiments, the albumin is human serum albumin. In some embodiments, the domain III consists of amino acid residues 384 to 585 of human serum albumin. In some embodiments, the domain III of human serum albumin has the amino acid sequence set forth in SEQ ID NO: 1, wherein representative amino acid sequences of the DIIIa subunit and DIIIb subunit are set forth in SEQ ID NO: 2 and SEQ ID NO: 3, respectively. In some embodiments, the complex is formed by nucleophilic substitution of the domain III of albumin with reactive groups (e.g., Cl⁻ or Br⁻) on the dye molecule . In some embodiments, the molar ratio between the domain III or variant thereof and the dye molecule ranges from 1:5 to 5:1 (e.g., 1:1).

In the present invention, variants include moieties, fusion recombinant proteins, and repeated moieties. In some embodiments, the small molecule is selected from one or more of a photosensitizer (e.g., a dye molecule, such as a cyanine dye molecule), a nuclide, and a small-molecule drug (e.g., a small-molecule chemotherapeutic agent).

In the present invention, a small molecule refers to a molecule having a molecular weight not greater than 500.

The albumin variant may be expressed in prokaryotic or eukaryotic cells, so that it possesses the biocompatibility of wild-type albumin. Organic dyes with near-infrared emission and high quantum yield often doped with heavy metal components, which may lead to long-term cytotoxicity. In contrast, the complex of the present invention significantly reduces the toxicity of drugs to the organism.

The small molecule contained in the complex may include reactive groups (e.g., Br or Cl), so as to enable binding to the albumin. In some embodiments, the reactive groups of the small molecule react with linking groups (e.g., mercapto group, thiol, hydroxyl group, carboxyl group or amino group) of serum albumin, thereby forming a linkage between the small molecule and the serum albumin. In some embodiments, the linking group of serum albumin is a thiol group. Accordingly, in some embodiments, the small molecule may form a covalent bond with the thiol group of serum albumin.

In some embodiments, the small molecule is a cyanine dye molecule.

In some embodiments, the dye molecule has one of the following backbone structures or derivatives thereof:
Skeleton 1 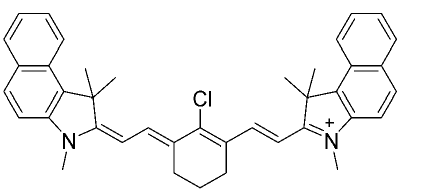
Skeleton 2 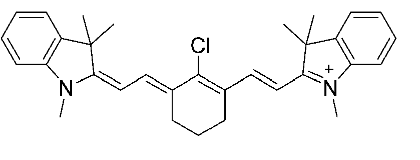
Skeleton 3 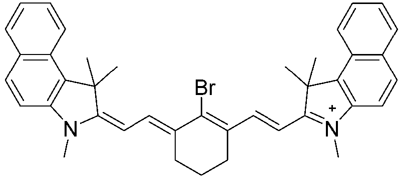
Skeleton 4

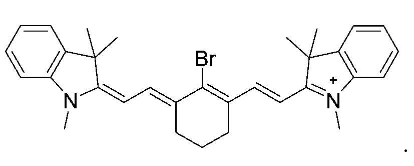

In some embodiments, the dye molecule has one of the following structures: 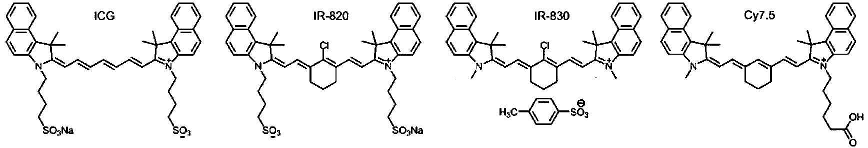 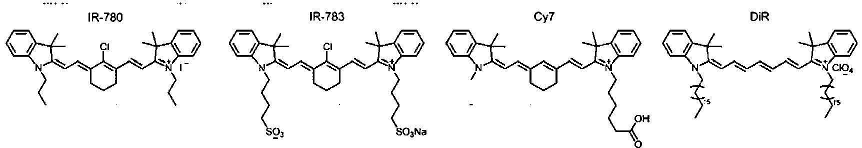 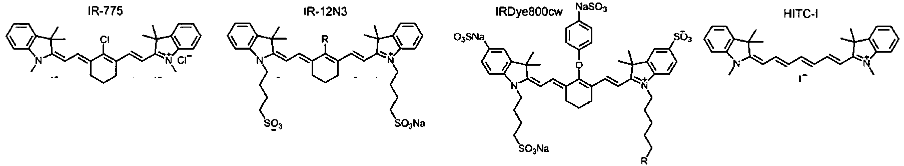 or a structure obtained by substituting the Cl atom in the backbone with F, Br, or I. 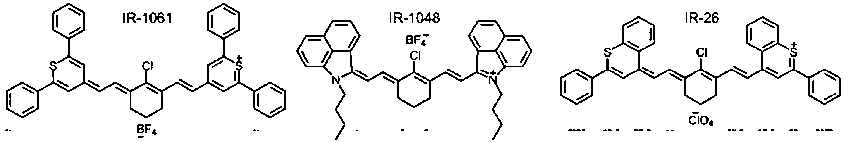 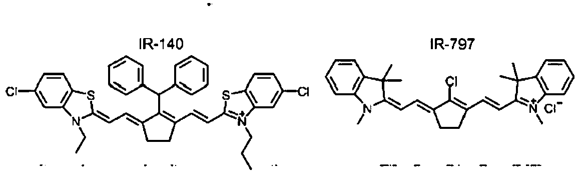 or a structure obtained by substituting the Cl atom in the backbone with F, Br, or I.

In some embodiments, the cyanine dye molecule contained in the complex is IR-780.

The functional moiety contained in the complex of the present invention may be a small-molecule compound that can be used for diagnosis, prevention, or treatment of a disease associated with a renal-bladder clearance pathway. Alternatively, the moiety may be a functional peptide applicable to such disease, which may be a linear peptide or a cyclic peptide, or a therapeutic protein for treating a kidney or bladder disease.

In some embodiments, the domain III of albumin or a variant thereof, or the DIIIa subunit or a variant thereof is linked with the functionalized moiety to form a fusion/recombinant protein, thereby obtaining a functionalized protein shell.

In some embodiments, the functionalized moiety is conjugated to the protein shell via a chemical compound, an amino acid, or a peptide. In some embodiments, the chemical compound is a small-molecule coupling agent for chemical conjugation (e.g., dibenzocyclooctyne-N-hydroxysuccinimidyl ester), which may also be replaced with any linker capable of effectively connecting the protein shell and the functionalized moiety. In other embodiments, the protein shell and the functional peptide or the therapeutic protein may be prepared as a fusion protein via genetic engineering (e.g., through eukaryotic or prokaryotic expression). For instance, fusion proteins can be expressed using eukaryotic or prokaryotic expression vectors (e.g., *Pichia pastoris*, *Escherichia coli*, or 293HT cells).

In some embodiments, the functionalized moiety may be a short peptide targeting bladder cancer, such as PLZ4 or its cyclic peptide, wherein the amino acid sequence of PLZ4 is QDGRMGF (SEQ ID NO: 10), and the amino acid sequence of the cyclic peptide (cPLZ4) is cQDGRMGFc (SEQ ID NO: 15); or may be other short peptides and derivatives thereof that target bladder cancer, including RGD or a fusion protein thereof, prostate-specific membrane antigen (PSMA), as well as other small molecules.

In some embodiments, the functionalized moiety is the EphB4-EBD protein (also referred to as EphB4 in some embodiments), another protein or small molecule having therapeutic effects on bladder cancer.

EphB4, also known as tyrosine kinase receptor B4, is a member of the Eph tyrosine kinase receptor family. It interacts with its ligand EphrinB2 to induce signaling pathways that play a key role in angiogenesis. The structure of EphB4 consists of extracellular, transmembrane, and intracellular domains. The extracellular domain of EphB4 is located outside the cell and mainly includes three parts: (1) an Ephrin-binding domain (EBD), which is a globular domain at the N-terminus responsible for binding to EphrinB2; (2) a Cys rich domain; and (3) two fibronectin type III-like domains. The intracellular domain of the EphB4 receptor is a tyrosine kinase active region, which includes the tyrosine kinase (TK) domain, the sterile alpha motif (SAM) domain, and the PDZ domain. This region facilitates autophosphorylation of its own tyrosine residues, thereby enhancing enzymatic activity, and subsequently catalyzes the phosphorylation of various intracellular substrate proteins, activating intracellular protein kinases and thus transmitting intracellular signals to the extracellular environment. The EphB4 receptor can bind to the EphrinB2 ligand via its extracellular domain, inducing phosphorylation of the intracellular tyrosine kinase active region, initiating signal transduction that promotes endothelial cell adhesion and migration, inhibits multiple growth factors in vivo, and promotes angiogenesis. Soluble EphB4 (sEphB4) is a soluble extracellular domain fragment of the EphB4 protein that binds to the EphrinB2 ligand, thereby blocking activation of the EphB4/EphrinB2 signaling pathway and inhibiting tumor angiogenesis.

As described above, the extracellular domain of the EPH receptor B4 (EphB4) consists of three components: the ephrin binding domain (EBD), a globular domain that binds to the ephrinB2 ligand; a Cys rich domain; and two fibronectin type III-like domains. The EphB4-EBD protein can be conjugated to the protein shell, and ultimately, the DIII-EphB4-EBD fusion protein can be expressed and purified via genetic engineering. The DIII-EphB4-EBD protein is a small-molecule protein that can form covalent conjugation with cyanine dye molecules and undergoes drug metabolism via the renal clearance pathway. This DIII-EphB4-EBD protein can serve as an antagonist to block the signaling pathway between EphB4 and its ligand EphrinB2, thereby inhibiting tumor angiogenesis. Meanwhile, the covalent complex formed by the DIII-EphB4-EBD recombinant protein and a cyanine dye molecule can also be applied for imaging and therapy of a disease affecting a major organ and tissue involved in the kidney-bladder metabolic pathway.

The EphB4 (EPH receptor B4) receptor protein described in the present invention may be of human origin, wherein the EphB4-EBD protein may be the extracellular domain of EphB4 and a subunit and a variant thereof, or the globular domain that binds to the ephrinB2 ligand, as well as a variant thereof such as a fragment, mutant, edited/modified product, or a repeated fragment.

An exemplary amino acid sequence of full-length human EphB4 protein is set forth in SEQ ID NO: 11.

An exemplary amino acid sequence of the EphB4-EBD protein is set forth in SEQ ID NO: 12.

In some embodiments, in the complex of the present invention, the protein shell and the functionalized moiety form a fusion/recombinant protein. In some embodiments, the functionalized moiety is selected from a short peptide PLZ4, a cyclic peptide cPLZ4, RGD, or an EphB4-EBD protein.

In some embodiments, the fusion protein is (DIII)ₐ₋(RGD)_{b}, wherein a and b are each independently an integer ≥1 (e.g., 1, 2, 3, 4, 5, 6, 7, 8, 9, or 10).

In some embodiments, the fusion/recombinant protein comprises an amino acid sequence selected from SEQ ID NO: 13, SEQ ID NO: 14, SEQ ID NO: 16, SEQ ID NO: 18, SEQ ID NO: 19, and SEQ ID NO: 20. Furthermore, the complex of the present invention (e.g., on the protein shell or the functionalized moiety) may be labeled with a radionuclide to enable early imaging and therapy of a disease. The radionuclide may be an imaging radionuclide, such as Cu-64 or F-18, or a therapeutic radionuclide, such as Lu-177, Y-90, I-131, I-125, Ac-225, or P-32.

In one aspect, the present application also provides a method for preparing a functionalized protein shell, comprising the following steps:
Step S1: preparing solution A comprising a domain III of albumin or a variant thereof, or a DIIIa subunit of albumin or a variant thereof;
Step S2: subjecting solution A to ultrafiltration centrifugation using an ultrafiltration tube, discarding filtrate, adding an alkaline solution (e.g., sodium bicarbonate solution), and vortexing at room temperature to adjust pH to an alkaline condition, thereby obtaining solution B;
Step S3: mixing a small-molecule coupling agent for chemical conjugation (e.g., dibenzocyclooctyne-N-hydroxysuccinimidyl ester) with solution B by vortexing at room temperature, allowing a reaction to proceed at room temperature or under heating, removing excess coupling agent by ultrafiltration centrifugation, and dissolving a product in phosphate-buffered saline (PBS) to obtain Compound 1;
Step S4: mixing Compound 1 with a raw material of the functionalized moiety (e.g., a short peptide stock solution) by vortexing at room temperature, allowing a reaction to proceed overnight at room temperature or under heating condition, removing excess functionalized moiety (e.g., short peptide) by ultrafiltration centrifugation, and finally dissolving a product in phosphate buffer (PBS) to obtain Compound 2.

In some embodiments, in Step S1, solution A is prepared by dissolving the domain III of albumin or a variant thereof, or a DIIIa subunit of albumin or a variant thereof, in phosphate-buffered saline (PBS) or purified water to achieve desired concentrations.

In some embodiments, in Step S2, the ultrafiltration tube has a molecular weight cutoff of 1 kDa to 20 kDa, more preferably 10 kDa; the centrifugation is performed at speed of 1000 g to 12,000 g, more preferably 4,000 g; the centrifugation is performed for 10 min to 120 min, more preferably 40 min; the centrifugation is performed at temperature of 0°C to 60°C, more preferably 4°C; the pH is in the range of 7.8 to 10, more preferably 8.3; and the vortex mixing is performed for 5 s to 60 s, more preferably 10 s.

In some embodiments, in Step S3, the coupling agent is prepared by dissolution in dimethyl sulfoxide (DMSO) and stored at -20°C until use; the coupling agent and solution B have a molar ratio of 0.01:1 to 1:10, more preferably 0.3:1; the vortex mixing is performed for5 s to 60 s, more preferably 10 s; the reaction is performed at temperature in the range of 20°C to 50°C, more preferably 37°C; the reaction is performed for 30 min to 180 min, more preferably 60 min; the ultrafiltration is performed with a ultrafiltration tube having a molecular weight cutoff of 1 kDa to 20 kDa, more preferably 10 kDa; the centrifugation is performed at speed from 1000 g to 12,000 g, more preferably 4,000 g; the centrifugation is performed for 10 min to 120 min, more preferably 40 min; and the centrifugation is performed at temperature of 0°C to 60°C, more preferably 4°C.

In some embodiments, in Step S4, the short peptide can be any functional peptide applicable to a disease involving the kidney-bladder clearance pathway, and may be a linear or cyclic peptide. The present invention takes bladder cancer as an example, using the short peptide cPLZ4 (amino acid sequence: cQDGRMGFc) that targets bladder cancer. The short peptide stock solution is prepared by dissolution in purified water or phosphate buffer (PBS) and stored at -20°C until use. The Compound 1 and the short peptide have a molar ratio of 0.5:1 to 1:1000, more preferably 1:100; the vortex mixing is performed for 5 s to 60 s, more preferably 10 s; the heating condition has a temperature of 20°C to 50°C, more preferably 37°C; the overnight reaction is performed for 6 h to 14 h, more preferably 12 h; the ultrafiltration is performed with a ultrafiltration tube having a molecular weight cutoff of 1 kDa to 20 kDa, more preferably 10 kDa; the centrifugation is performed at speed of 1000 g to 12,000 g, more preferably 4,000 g; the centrifugation is performed for 10 min to 120 min, more preferably 40 min; and the centrifugation is performed at temperature of 0°C to 60°C, more preferably 4°C.

The present invention also provides a method for preparing the complex, comprising the following steps:
S1: preparing solution A comprising a functionalized protein shell, and preparing solution B comprising a small molecule, such as a photosensitizer, for example, a dye molecule (e.g., a cyanine dye molecule), a radionuclide, or a small molecule drug (e.g., a small molecule chemotherapeutic agent).
S2: mixing solution A and solution B by vortexing at room temperature, and obtaining the complex at room temperature or under heating condition.

In some embodiments, in Step S1, solution A is prepared by dissolving the functionalized protein shell in phosphate-buffered saline (PBS) or purified water to achieve desired concentrations; the small molecule stock solution for solution B is prepared by dissolution in dimethyl sulfoxide (DMSO) and stored at -20°C until use.

In some embodiments, in Step S2, the vortex mixing is performed for 5 s to 60 s, more preferably 10 s; the heating condition has a temperature of 20°C to 80°C, more preferably 60°C; the heating condition has a time of 5 min to 120 min, more preferably 10 min; the solution A and solution B has a molar ratio of 0.01:1 to 1:100, more preferably 1:1.

In one aspect, the present application provides a use of the complex of the present invention as a fluorescent probe.

In one aspect, the present application provides a pharmaceutical composition comprising the complex of the present invention, and optionally one or more pharmaceutically acceptable carriers or excipients. The pharmaceutical composition of the present invention may be administered by any suitable route, including intravenous injection, intravesical administration, intratumoral injection, or intraperitoneal injection. The complex of the present invention can be used for near-infrared fluorescence imaging, as well as for in vivo photodynamic-mediated tumor killing . Accordingly, in some embodiments, the pharmaceutical composition is a contrast agent, and in other embodiments, the pharmaceutical composition is a therapeutic agent.

The complex provided by the present invention has a relatively small molecular weight. Additionally, the third domain of albumin (DIII) can form a stable covalent conjugation with the small molecule, allowing the complex to be metabolized to the bladder via the renal clearance pathway. Therefore, it can be administered via intravenous injection for the treatment of diseases affecting the major organs and tissues involved in the kidney-bladder metabolic pathway.

In another aspect, the present application provides a method for diagnosing, preventing, or treating a urological disease, the method comprising administering the complex of the present invention to a subject in need thereof. The disease includes, but is not limited to, urological tumors, such as renal cancer, Wilms tumor, bladder rhabdomyosarcoma, renal pelvic/ureteral cancer, bladder cancer, urethral cancer, or upper tract urothelial carcinoma; and urological injuries, such as renal injury. The disease may be a disease affecting the major organs and tissues involved in the kidney-bladder metabolic pathway. The method includes, but is not limited to, photodynamic therapy, imaging, surgical navigation, radiotherapy, or chemotherapy.

In another aspect, the present application provides a use of the complex of the present invention for preparation of a medicament for diagnosing, preventing, or treating a urological disease. The disease includes, but is not limited to, urological tumors (e.g., the tumors described above) and urological injuries (e.g., renal injury). The disease may be a disease occurring in major organs and tissues involved in the kidney-bladder metabolic pathway.

In one aspect, the present application provides a kit comprising the complex of the present invention; optionally, the kit further comprises one or more selected from the group consisting of: an antibody, a primer, a reagent for fixing and/or permeabilizing cells, or any combination thereof.

In one aspect, the present application provides a method for targeting a cell, the method comprising contacting the cell with the complex as described above; optionally, after the contacting, the cell is subjected to laser irradiation to obtain imaging of the cell.

In one aspect, the present application provides a use of the complex as described above for preparation of a kit, wherein the kit is used for targeting a cell or for obtaining imaging of a cell.

In some embodiments, the complex targets the cell by binding to a cell surface molecule, a cell surface protein, or a cell surface receptor expressed on the cell.

In some embodiments, the cells are located in the urinary system, for example, the kidney, ureter, bladder, or urethra. In some embodiments, the cells are tumor cells. The tumor may be a tumor occurring in any part of the urinary system, including but not limited to renal cancer, Wilms tumor, bladder rhabdomyosarcoma, renal pelvic/ureteral cancer, bladder cancer, urethral cancer, or upper tract urothelial carcinoma.

In some embodiments, the cells are present in tissues or in a living subject.

In another aspect, the present application provides an imaging method, the method comprising using the complex as described above as an imaging agent.

In some embodiments, the imaging is near-infrared region I (NIR-I) and/or near-infrared region II (NIR-II) fluorescence imaging.

In some embodiments, the imaging method is fluorescence imaging of cells, tissues, or a living subject.

### Definitions

Unless otherwise specified, the scientific and technical terms used herein have the meanings commonly understood by a person skilled in the art. In addition, the molecular genetics, nucleic acid chemistry, chemistry, molecular biology, biochemistry, cell culture, microbiology, cell biology, genomics, and recombinant DNA operations described herein are conventional techniques widely used in the relevant fields. In order to better understand the present invention, definitions and explanations of related terms are provided below.

As used herein, the term "wild-type human serum albumin" refers to naturally occurring human serum albumin having biological activity. The amino acid sequence of wild-type human serum albumin can be conveniently obtained from various public databases (e.g., the GenBank database). In some embodiments, the GenBank accession number of the human serum albumin is AEE60908.1.

As used herein, the term "cysteine" (also abbreviated as "Cys") refers to a common amino acid found in living organisms. Cysteine is the only amino acid among the more than 20 amino acids constituting proteins that contains a reducible sulfhydryl (-SH) group.

As used herein, the term "sulfhydryl group," also known as a thiol group or a mercapto group, refers to a monovalent functional group composed of one sulfur atom and one hydrogen atom, having the chemical formula -SH.

As used herein, the term "tertiary structure of a protein" refers to the regular three-dimensional spatial structure formed by further folding or coiling of a polypeptide chain on the basis of its various secondary structures.

As used herein, the term "quaternary structure of a protein" refers to the spatial structure formed by the association of two or more polypeptide chains, each having an independent tertiary structure, which are assembled through secondary interactions.

As used herein, the term "subunit" refers to each polypeptide chain having an independent tertiary structure in a protein with a quaternary structure.

As used herein, the term "domain" refers to a basic unit constituting the tertiary structure of a protein and having a distinct three-dimensional conformation. Different domains of a protein are typically distinguishable in space. In some embodiments, when a protein comprises multiple polypeptide chains, the domains of the protein may comprise multiple subunits.

### Advantages of the Invention

Compared with the prior technique, the present invention has the following advantages:
1. The complex of the present invention has a relatively small molecular weight and size, and thus can be administered via intravenous injection to treat diseases of major organs and tissues involved in the kidney-bladder metabolic pathway. For example, compared with invasive intravesical instillation, intravenous administration avoids the pain associated with cystoscopy or catheter insertion through the urethra. In addition, cystoscopic examination is relatively expensive, and intravenous administration can reduce the economic burden on patients to a certain extent.
2. The complex of the present invention, which comprises a photosensitizer such as a cyanine dye, exhibits superior photostability, high photoconversion efficiency, high quantum yield, and a significantly extended emission wavelength compared with near-infrared fluorescent probes used clinically. Accordingly, it is suitable for deep tissue imaging and photodynamic therapy, and can effectively be used for photodynamic treatment of diseases in major organs and tissues involved in the kidney-bladder metabolic pathway. For patients with high-risk NMIBC, not only is there a substantial economic burden due to the high cost of treatment, but the efficacy of chemotherapeutic agents (e.g., Bacillus Calmette-Guérin (BCG), a first-line therapy in clinical practice) remains limited. Apart from radical cystectomy, there are few effective salvage treatment options. The present invention undoubtedly provides a favorable alternative therapeutic option for patients with BCG-refractory high-risk NMIBC.
3. In the complex of the present invention, the small molecule may also be a radionuclide, a small molecule chemotherapeutic agent, etc. Therefore, the complex of the present invention enables the combined use of multiple therapeutic modalities, including radiotherapy, chemotherapy, and photodynamic therapy.

The embodiments of the present invention will be described in detail below with reference to the accompanying drawings and examples. However, those skilled in the art will appreciate that the drawings and examples are provided for illustrative purposes only and are not intended to limit the scope of the present invention. Various objects and advantageous features of the present invention will become apparent to those skilled in the art from the following detailed description of the drawings and preferred embodiments.

### Brief Description of the Drawings

Fig. 1 shows the mass spectrum and electrophoretogram of the protein shell (DIII-cPLZ4) obtained by functionalizing a variant of the DIII of albumin via chemical modification in Example 1 of the present invention.
Fig. 2 shows the absorption and emission spectra of the complex Cyanine@cDIII-PLZ4 described in Example 1 of the present invention.
Fig. 3 is a fluorescence enhancement diagram of the cyanine dye after binding to the functionalized variant of DIII of albumin via chemical modification described in Example 1 of the present invention.
Fig. 4 shows the electrophoretic analysis of the fusion protein DIII-PLZ4 expressed by genetic engineering (Fig. 4A), and the fluorescence enhancement after binding with the cyanine dye (Figs. 4B-4C) described in Example 2 of the present invention.
Fig. 5 shows the photostability of the complex in phosphate-buffered saline (PBS) and mouse urine described in Example 2 of the present invention.
Fig. 6 presents a comparison of the pharmacokinetics of the complex and free cyanine dye in mice described in Example 3 of the present invention.
Fig. 7 shows upright fluorescence microscopy and flow cytometry results illustrating the uptake of different drugs by different cells in Example 4 of the present invention.
Fig. 8 shows the validation of the target binding site of the complex to bladder cancer cells in Example 4 of the present invention.
Fig. 9 shows the cytotoxicity of the complex against bladder cancer cells at different concentrations and light doses described in Example 5 of the present invention.
Fig. 10 shows confocal microscopy and flow cytometry results of the complex for monitoring photodynamic-mediated reactive oxygen species generation in bladder cancer cells described in Example 6 of the present invention.
Fig. 11 illustrates the establishment of an orthotopic bladder cancer mouse model as described in Example 7 of the present invention.
Fig. 12 shows the fluorescence accumulation of the complex in orthotopic bladder cancer-bearing mice described in Example 8 of the present invention.
Fig. 13 shows the photodynamic-mediated tumor killing with the complex and post-treatment survival monitoring in BALB/c orthotopic bladder cancer-bearing mice described in Example 9 of the present invention.
Fig. 14 shows the photodynamic-mediated tumor killing with the complex and post-treatment survival monitoring in C57BL/6 orthotopic bladder cancer-bearing mice described in Example 10 of the present invention.
Fig. 15 shows the toxicity of the complex in healthy mice described in Example 11 of the present invention.
Fig. 16 shows the radionuclide labeling with ⁶⁴Cu and preliminary evaluation.
Fig. 17 shows the radionuclide labeling with ¹⁷⁷Lu and preliminary evaluation.
Fig. 18 shows the photodynamic therapy efficacy of DIII fusion-expressed tumor-targeting peptide (DIII-tar pep) administered via intravenous injection (i.v.) or intravesical infusion.
Fig. 19 shows the yeast expression of the EphB4-DIII fusion protein and its binding evaluation with a photosensitizer.
Fig. 20 shows the expression of DIII-RGD and (DIII)m-(RGD)n and their binding evaluation with a photosensitizer.
Fig. 21 shows the targeting specificity of DIII-RGD and (DIII)m-(RGD)n to three different tumor cell lines.
Fig. 22 shows the in vivo evaluation of DIII-RGD and (DIII)m-(RGD)n.

### Sequence Information

The information for certain sequences referred to in the present invention is provided in Table 1 below.

**Table 1: Description of Sequences**

| SEQ ID NO: | Description | Sequences |
|---|---|---|
| 1 | Domain III(DIII)of albumin | VEEPQNLIKQNCELFEQLGEYKFQNALLVRYTKKVPQV STPTLVEVSRNLGKVGSKCCKHPEAKRMPCAEDYLSV VLNQLCVLHEKTPVSDRVTKCCTESLVNRRPCFSALEV DETYVPKEFNAETFTFHADICTLSEKERQIKKQTALVEL VKHKPKATKEQLKAVMDDFFVEKCCKADDKETCFAEE GKKLVSQLGL |
| 2 | DIIIa subunit of albumin | KCCADPHECYAKVFDEFKPLVEEPQNLIKQNCELFEQL GEYKFQNALLVRYTKKVPQVSTPTLVEVSRNLGKVGSK CCKHPEAKRMPCAEDYLSVVLNQLCVLHEKTPVSDRV TKCCTESLVNRRPCFSALEVDETYVPKEFNAETFTFHAD ICTLSEKERQIKKQTALVELVKHKPKATKEQLKAVMDD FFVEKCCKA |
| 3 | DIIIb subunit of albumin | KTPVSDRVTKCCTESLVNRRPCFSALEVDETYVPKEFN AETFTFHADICTLSEKERQIKKQTALVELVKHKPKATKE QLKAVMDDFFVEKCCKADDKETCFAEEGKKLVSQLGL |
| 4 | HA | YPYDVPDYA |
| 5 | His | HHHHHH |
| 6 | GST | MSPILGYWKIKGLVQPTRLLLEYLEEKYEEHLYERDEG DKWRNKKFELGLEFPNLPYYIDGDVKLTQSMAIIRYIAD KHNMLGGCPKERAEISMLEGAVLDIRYGVSRIAYSKDF ETLKVDFLSKLPEMLKMFEDRLCHK TYLNGDHVTHPDFMLYDALD VVLYMDPMCLDAFPKLVCFKKRIEAIPQIDKYLKSSKYI AWPLQGWQATFGGGDHPPK |
| 7 | Myc | EQKLISEEDL |
| 8 | Linker 1 | GGGGS |
| 9 | Linker 2 | GGGGSGGGGS |
| 10 | PLZ4 | CQDGRMGFC |
| 11 | Full-length human EphB4 protein | |
| | | |
| 12 | EphB4-EBD | EETLLNTKLETADLKWVTFPQVDGQWEELSGLDEEQH SVRTYEVCDVQRAPGQAHWLRTGWVPRRGAVHVYAT LRFTMLECLSLPRAGRSCKETFTVFYYESDADTATALTP AWMENPYIKVDTVAAEHLTRKRPGAEATGKVNVKTLR LGPLSKAGFYLAFQDQGACMALLSLHLFYKKCAQLTV |
| 13 | DIII-PLZ4 | VEEPQNLIKQNCELFEQLGEYKFQNALLVRYTKKVPQV STPTLVEVSRNLGKVGSKCCKHPEAKRMPCAEDYLSV VLNQLCVLHEKTPVSDRVTKCCTESLVNRRPCFSALEV DETYVPKEFNAETFTFHADICTLSEKERQIKKQTALVEL VKHKPKATKEQLKAVMDDFFVEKCCKADDKETCFAEE GKKLVSQLGLGGGGSQDGRMGF |
| 14 | DIII-EphB4-EBD | VEEPQNLIKQNCELFEQLGEYKFQNALLVRYTKKVPQV STPTLVEVSRNLGKVGSKCCKHPEAKRMPCAEDYLSV VLNQLCVLHEKTPVSDRVTKCCTESLVNRRPCFSALEV DETYVPKEFNAETFTFHADICTLSEKERQIKKQTALVEL VKHKPKATKEQLKAVMDDFFVEKCCKADDKETCFAEE GKKLVSQLGLGGGGSGGGGSEETLLNTKLETADLKWV TFPQVDGQWEELSGLDEEQHSVRTYEVCDVQRAPGQA HWLRTGWVPRRGAVHVYATLRFTMLECLSLPRAGRSC KETFTVFYYESDADTATALTPAWMENPYIKVDTVAAEH LTRKRPGAEATGKVNVKTLRLGPLSKAGFYLAFQDQG ACMALLSLHLFYKKCAQLTV |
| 15 | cPLZ4 | cQDGRMGFc |
| 16 | DIII-cPLZ4 | VEEPQNLIKQNCELFEQLGEYKFQNALLVRYTKKVPQV STPTLVEVSRNLGKVGSKCCKHPEAKRMPCAEDYLSV VLNQLCVLHEKTPVSDRVTKCCTESLVNRRPCFSALEV DETYVPKEFNAETFTFHADICTLSEKERQIKKQTALVEL VKHKPKATKEQLKAVMDDFFVEKCCKADDKETCFAEE GKKLVSQLGLGGGGSCQDGRMGFC |
| 17 | RGD | RGD |
| 18 | DIII-RGD | VEEPQNLIKQNCELFEQLGEYKFQNALLVRYTKKVPQV STPTLVEVSRNLGKVGSKCCKHPEAKRMPCAEDYLSV VLNQLCVLHEKTPVSDRVTKCCTESLVNRRPCFSALEV DETYVPKEFNAETFTFHADICTLSEKERQIKKQTALVEL VKHKPKATKEQLKAVMDDFAAFVEKCCKADDKETCFA EEGKKLVAASQAALGLGGGGSRGD |
| 19 | RGD-DIII-RGD | RGDGGGGSVEEPQNLIKQNCELFEQLGEYKFQNALLVR YTKKVPQVSTPTLVEVSRNLGKVGSKCCKHPEAKRMP CAEDYLSVVLNQLCVLHEKTPVSDRVTKCCTESLVNRR PCFSALEVDETYVPKEFNAETFTFHADICTLSEKERQIK KQTALVELVKHKPKATKEQLKAVMDDFAAFVEKCCKA DDKETCFAEEGKKLVAASQAALGLGGGGSRGD |
| 20 | RGD-DIII-RGD-DIII-RGD | RGDGGGGSVEEPQNLIKQNCELFEQLGEYKFQNALLVR YTKKVPQVSTPTLVEVSRNLGKVGSKCCKHPEAKRMP CAEDYLSVVLNQLCVLHEKTPVSDRVTKCCTESLVNRR PCFSALEVDETYVPKEFNAETFTFHADICTLSEKERQIK KQTALVELVKHKPKATKEQLKAVMDDFAAFVEKCCKA DDKETCFAEEGKKLVAASQAALGLGGGGSRGDGGGGS VEEPQNLIKQNCELFEQLGEYKFQNALLVRYTKKVPQV STPTLVEVSRNLGKVGSKCCKHPEAKRMPCAEDYLSV VLNQLCVLHEKTPVSDRVTKCCTESLVNRRPCFSALEV DETYVPKEFNAETFTFHADICTLSEKERQIKKQTALVEL VKHKPKATKEQLKAVMDDFAAFVEKCCKADDKETCFA EEGKKLVAASQAALGLGGGGSRGD |

### Specific Models for Carrying Out the present invention

The present invention is described below with reference to the following examples, which are intended to illustrate, but not to limit, the present invention.

Unless otherwise specified, the experiments and methods described in the examples were performed essentially in accordance with conventional methods well known in the art and described in various references. For example, conventional techniques in immunology, biochemistry, chemistry, molecular biology, microbiology, cell biology, genomics, and recombinant DNA used in the present invention can be found in Sambrook, Fritsch, and Maniatis, MOLECULAR CLONING:A LABORATORY MANUAL, 2nd Edition (1989); CURRENT PROTOCOLS IN MOLECULAR BIOLOGY (edited by F.M. Ausubel et al., (1987)); the METHODS IN ENZYMOLOGY series (Academic Press); PCR 2: A PRACTICAL APPROACH (edited by M.J. MacPherson, B.D. Hames, and G.R. Taylor (1995)); and (ANIMAL CELL CULTURE edited by R.I. Freshney (1987)).

Additionally, unless otherwise specified in the examples, all experiments were performed under conventional conditions or under conditions recommended by the manufacturer. Reagents or instruments without specified manufacturers are conventional products commercially available. Those skilled in the art will appreciate that the examples are provided for illustrative purposes only and are not intended to limit the scope of the claimed invention. All publications and other references mentioned herein are incorporated by reference in their entirety.

### Example 1: Preparation of the Complex Cyanine@DIII-cPLZ4

The complex of the present invention is formed by covalent conjugation of the domain III of albumin or a variant thereof with a dye molecule. The core chromophore is the dye molecule, and the moiety covalently conjugated to the exterior of the core chromophore is the albumin variant. The protein shell of the probe can be functionalized through genetic engineering or chemical conjugation.

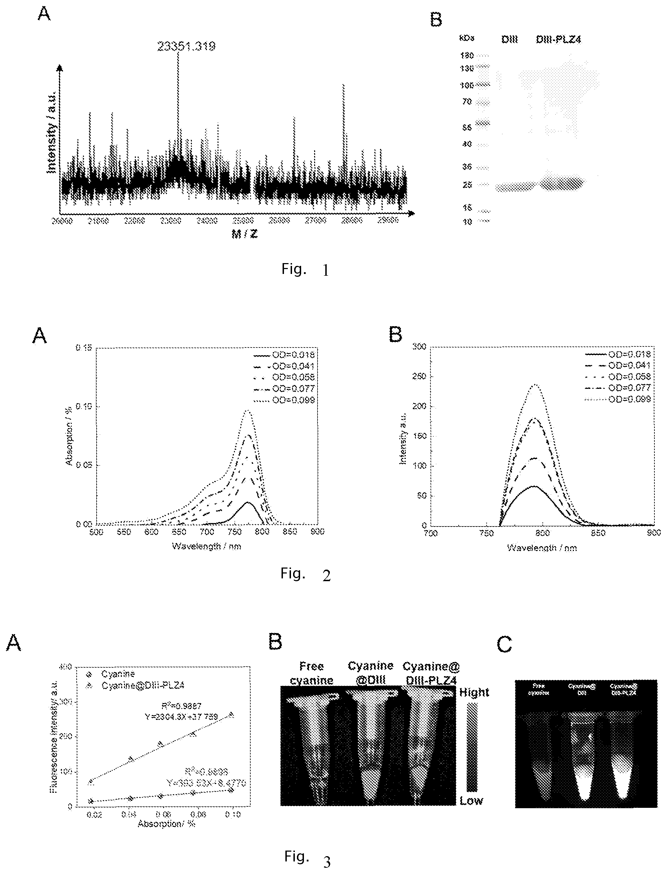

In this embodiment, the functionalized protein shell moiety DIII-cPLZ4 is obtained by chemically linking the domain III of albumin with cyclic PLZ4 (cPLZ4, amino acid sequence: cQDGRMGFc), which is a cyclic peptide of the specific targeting short peptide PLZ4. The chemical preparation method thereof comprises the following specific steps:
Solution A of the domain III of albumin was prepared (85 µM, 100 µL). Solution A was subjected to ultrafiltration centrifugation at 4°C using a 10 kDa ultrafiltration tube, and the filtrate was discarded. A 0.1 M sodium bicarbonate solution was then added, followed by vortex mixing at room temperature for 10 s to adjust the pH to 8.3, thereby obtaining solution B. Then, 2.565 µL of 10 mM dibenzocyclooctyne-N-hydroxysuccinimide ester (DBCO-NHS) was mixed with solution B by vortexing at room temperature for 10 s, followed by reaction at 37°C for 1 h. Excess dibenzocyclooctyne-N-hydroxysuccinimide ester was removed by ultrafiltration centrifugation at 4°C using a 10 kDa ultrafiltration tube, and the product was dissolved in phosphate-buffered saline (PBS) and brought to a final volume of 100 µL to obtain Compound 1. Compound 1 was then mixed with 3 µL of an 8.55 mM stock solution of cPLZ4 (amino acid sequence: cQDGRMGFc) by vortexing at room temperature for 10 s, followed by overnight reaction at 37°C for 12 h. Excess cPLZ4 was removed by ultrafiltration centrifugation at 4°C using a 10 kDa ultrafiltration tube. The product was finally dissolved in phosphate-buffered saline (PBS) to a total volume of 100 µL, thereby obtaining the compound, namely the functionalized protein shell DIII-cPLZ4c, the amino acid sequence of which is set forth in SEQ ID NO: 13. The mass spectrum (Fig. 1A) and the Western blot electrophoresis results (Fig. 1B) indicate that the molecular weight of DIII-cPLZ4 is about 23 kDa.

This example also provides a method for preparing the complex Cyanine@DIII-cPLZ4:
The functionalized protein shell DIII-cPLZ4 and the cyanine dye IR-780 were mixed at a molar ratio of 1:1 and heated at 60°C for 10 min. The resulting compound was the complex Cyanine@DIII-cPLZ4.

### Example 2: Fusion Recombinant Expression of the Cyanine@DIII-PLZ4 Complex

The complex of the present invention is formed by covalent binding of a fusion-expressed protein of the third domain of albumin (DIII) and the PLZ4 peptide with dye molecules, wherein the core chromophore is the dye molecule, and the portion covalently bound to the exterior of the core chromophore is the albumin fusion protein. The protein shell portion of the probe may be functionalized by means of genetic engineering modification or chemical grafting.

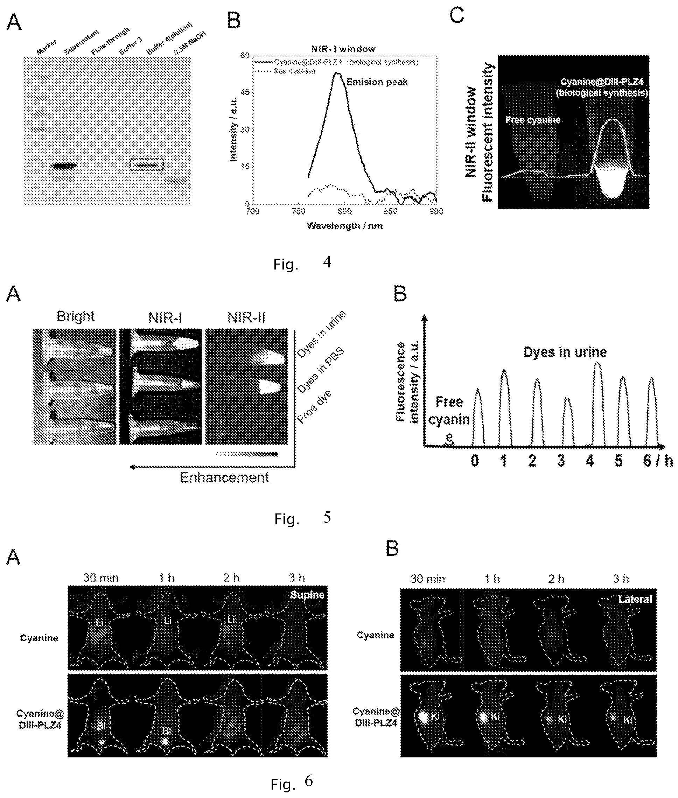

In this embodiment, the functionalized protein shell moiety DIII-PLZ4 is obtained from a fusion recombinant protein of domain DIII of albumin and the specific targeting short peptide PLZ4, and the fusion recombinant protein is covalently bound to dye molecules. The preparation method thereof comprises the following specific steps:
The DIII-PLZ4 fusion protein was expressed using a eukaryotic/prokaryotic expression system (for example, *Pichia pastoris*, *Escherichia coli*, or 293HT cells). As confirmed by gel electrophoresis (Fig. 4A), the molecular weight of DIII-PLZ4 is about 23 kDa.

This embodiment further provides a method for preparing the complex dye@DIII-PLZ4:
The functionalized protein shell DIII-PLZ4 and the dye IR-783 were mixed at a molar ratio of 1:1 and then heated at 60°C for 10 min, and the resulting compound was the complex dye@DIII-PLZ4.

### Example 3: In Vitro Characterization of the Complexes Cyanine@DIII-cPLZ4 and Cyanine@DIII-PLZ4

The absorption and emission spectra of the complex Cyanine@DIII-cPLZ4 prepared in Example 1 at different gradient concentrations were measured, using phosphate-buffered saline (PBS) as a blank baseline. The results showed that the complex exhibited an absorption peak in the NIR-I region (Fig. 2A) and an emission peak in the NIR-I region (Fig. 2B). Mixing the cyanine dye with DIII-PLZ4 significantly enhanced the fluorescence intensity (Fig. 3A). Fluorescence imaging of the complex in the NIR-I region (Fig. 3B) and the NIR-II region (Fig. 3C) also confirmed the significant enhancement in fluorescence intensity.

Fig. 4 shows the electrophoretic profile of the fusion protein DIII-PLZ4 expressed by genetic engineering (Fig. 4A), as well as fluorescence enhancement images after binding with the cyanine dye (Figs. 4B-C).

The 85 µM complex Cyanine@DIII-PLZ4 prepared in Example 2 was diluted 28.3-fold to 3 µM with phosphate-buffered saline (PBS) and fresh mouse urine, respectively. The latter was incubated at 37°C for 1 h, 2 h, 3 h, 4 h, 5 h, and 6 h, respectively, followed by NIR-I and NIR-II imaging. The results showed that, in urine at physiological temperature, the fluorescence intensity of the complex did not decrease significantly (Fig. 5A), and no significant change in fluorescence intensity was observed even after incubation for up to 6 h (Fig. 5B).

### Example 4: Comparison of the In Vivo Pharmacokinetic Results of Cyanine@DIII-PLZ4 and Free Cyanine Dye in Mice

The 3 µM Cyanine@DIII-PLZ4 prepared in Example 2 and 3 µM free cyanine dye were separately administered into normal BALB/c mice with whole-body hair removal by tail vein injection. NIR-II fluorescence imaging of the front and lateral sides of the mice was performed at 30 min, 1 h, 2 h, and 3 h after administration. The results showed that Cyanine@DIII-PLZ4 could be metabolized to the bladder through the renal clearance pathway, and fluorescence accumulation in the bladder could be observed as early as 30 min, whereas the free cyanine dye was retained in the liver (Fig. 6).

### Example 5: Tumor Cell-Targeting Ability of Cyanine@DIII-cPLZ4

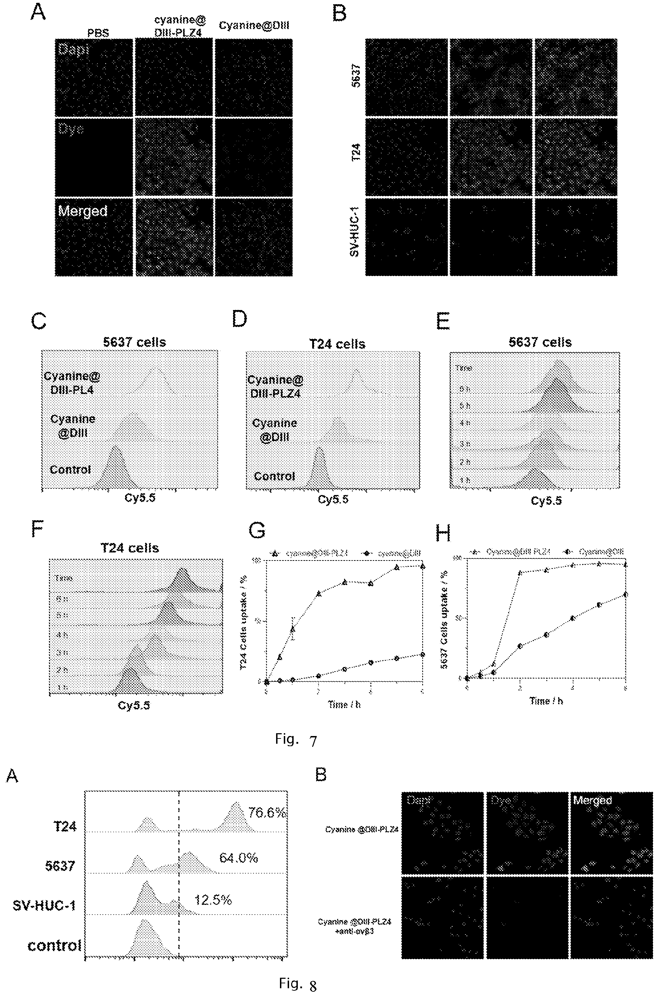

The targeting property of Cyanine@DIII-PLZ4 toward bladder cancer cells was observed using an upright fluorescence microscope. Human bladder cancer T24 cells were seeded in an 8-well chamber slide at a density of 1×10⁴ cells per well. After incubation in an incubator for 24 h, the culture medium was removed, and the cells were gently washed 2-3 times with sterile phosphate-buffered saline (PBS). Then, 100 µL of 4% paraformaldehyde was added to each well for fixation on ice for 10-20 min, followed by repeated washing with phosphate-buffered saline (PBS). Thereafter, 100 µL per well of each of the following three treatment groups was added, respectively: 1 µM Cyanine@DIII-PLZ4 diluted in serum-free medium, 1 µM Cyanine@DIII diluted in serum-free medium, and serum-free medium alone. After incubation for 10 min, the cells were repeatedly washed with PBS. Subsequently, one drop (about the size of a mung bean) of a DAPI reagent containing an anti-fluorescence quenching agent was added to each well, followed by coverslipping, and observation was carried out under an upright fluorescence microscope. Compared with the control groups, the Cyanine@DIII-PLZ4 group exhibited higher fluorescence targeting toward T24 cells (Fig. 7A). Two human bladder cancer cell lines, T24 and 5637, and one normal urothelial cell line, SV-HUC-1, were respectively seeded in three 8-well chamber slides at a density of 1×10⁴ cells per well. The remaining procedures were the same as described above, and the cells were finally observed under an upright fluorescence microscope. The results demonstrated that Cyanine@DIII-PLZ4 exhibited relatively high targeting ability toward the two human bladder cancer cell lines T24 and 5637, while exhibiting relatively low targeting ability toward the normal urothelial cell line SV-HUC-1 (Fig. 7B).

The uptake of Cyanine@DIII-PLZ4 by human bladder cancer cells was evaluated by flow cytometry. Two human bladder cancer cell lines were respectively seeded into a plurality of 6-well plates at a density of 2×10⁵ cells per well. After incubation for 24 h, the culture medium was removed, and the cells were gently washed 2-3 times with phosphate-buffered saline (PBS). The cells were then incubated with serum-free medium containing 3 µM Cyanine@DIII-PLZ4 or 3 µM Cyanine@DIII, respectively, at 37°C for 1 h, 2 h, 3 h, 4 h, 5 h, or 6 h. Three replicate wells were prepared for each time point, and a serum-free medium group was included as a control. After incubation, each well was washed 3-5 times with PBS to remove unbound drug. Trypsin was then added to each well to dissociate the adherent cells into single cells, followed by centrifugation at 1000 rpm for 3 min at room temperature. The cells were resuspended in serum-free medium and immediately analyzed by flow cytometry. The results showed that, compared with the control group, both human bladder cancer cell lines exhibited relatively high uptake of Cyanine@DIII-cPLZ4 (Figs. 7C-D and 7G-H). In addition, the uptake increased with increasing incubation time, and the uptake rate reached more than 90% after 6 h (Figs. 7E-F and 7G-H).

Further, it was verified that the targeting binding site of Cyanine@DIII-cPLZ4 on bladder cancer cells is the αvβ3 integrin receptor. First, the expression of the αvβ3 integrin receptor on the surfaces of two human bladder cancer cell lines, T24 and 5637, and one normal urothelial cell line, SV-HUC-1, was analyzed by flow cytometry. The three cell lines were respectively seeded in separate 6-well plates at a density of 2×10⁵ cells per well and cultured for 24 h. The adherent cells were digested with trypsin to obtain single-cell suspensions, followed by centrifugation at 1000 rpm for 3 min. The cells were resuspended in a serum blocking solution (a buffer solution containing 0.5% phosphoric acid) and incubated at room temperature for 20 min, followed by centrifugation at 1000 rpm for 3 min. A diluted primary antibody (1:100) was then added and incubated at 37°C for 2 h, and the supernatant was removed by centrifugation at 1000 rpm for 3 min. The cells were resuspended and washed with PBS for 5 min, followed by centrifugation at 1000 rpm for 3 min. The washing was repeated 3 times. A diluted secondary antibody (1:100) was added and incubated at 37°C for 30 min, and the supernatant was removed by centrifugation at 1000 rpm for 3 min. The cells were resuspended and washed with PBS for 5 min, followed by centrifugation at 1000 rpm for 3 min. The washing was repeated 3 times. As shown in Fig. 8A, the two human bladder cancer cell lines highly expressed αvβ3, whereas the normal urothelial cell line exhibited low expression. Next, T24 cells were seeded in an 8-well chamber slide at a density of 1×10⁴ cells per well. After incubation for 24 h, an anti-αvβ3 reagent was added and incubated at 37°C for 1 h, followed by washing three times with PBS for 5min each time. The cells were then fixed with 4% paraformaldehyde on ice for 10-20 min and repeatedly washed with PBS. Thereafter, 100 µL of 1 µM Cyanine@DIII-cPLZ4 diluted in serum-free medium was added and incubated for 10 min. After washing with PBS, one drop (about the size of a mung bean) of a DAPI reagent containing an anti-fluorescence quenching agent was added to each well, followed by coverslipping, and observation was carried out under an upright fluorescence microscope. The results showed that the tumor-targeting ability was substantially reduced in the anti-αvβ3 antibody-treated group (Fig. 8B), indicating that the αvβ3 integrin receptor is the binding site through which the complex targets tumor cells.

### Example 6: Cytotoxic Effect of the Complex on Bladder Tumor Cells at Different Concentrations and Light Doses

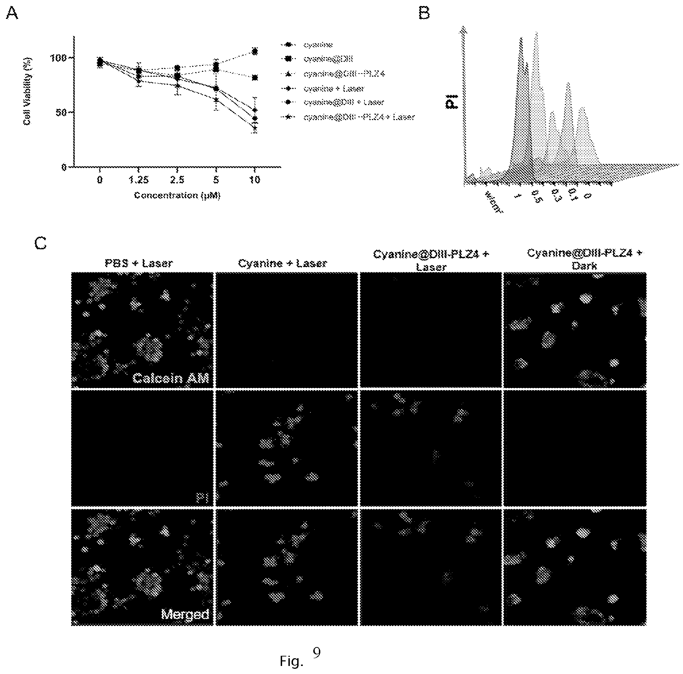

MB-49 cells were seeded in a 96-well plate at a density of 5,000 cells per well. After incubation for 24 h, the cells were washed twice with PBS. Six treatment groups were established, namely Cyanine+laser, Cyanine@DIII+laser, Cyanine@DIII-PLZ4+laser, Cyanine+dark, Cyanine@DIII+dark, and Cyanine@DIII-PLZ4+dark. Cyanine, Cyanine@DIII, and Cyanine@DIII-cPLZ4 were each diluted with serum-free medium to concentrations of 0, 1.25, 2.5, 5, and 10 µM. The cells were incubated with the respective drugs for 4 h, washed repeatedly with PBS to remove the drugs, and then replenished with serum-free medium. The laser groups were irradiated with an 808 nm laser for 8 min at a power density of 0.3 W/cm², while the non-laser groups were kept in the dark. After a further 12 h of incubation, the cells were incubated with CCK8 reagent diluted in serum-free medium for 30 min, and the results were measured using a microplate reader. The results showed that, compared with the control groups, the Cyanine@DIII-cPLZ4+laser group exhibited a significant cytotoxic effect, and the cytotoxicity increased with increasing drug concentration. In contrast, almost no cytotoxicity was observed in the groups without laser treatment, indicating that the complex has good biosafety (Fig. 9A).

MB-49 cells were seeded in a 96-well plate with 5,000 cells per well. After incubation for 24 h, the cells were washed twice with PBS. Cyanine@DIII-cPLZ4 was diluted in serum-free medium to a concentration of 5 µM, and the cells were incubated for 4 h. The cells were then washed with PBS repeatedly to remove the drug and replenished with serum-free medium. The laser groups were irradiated with an 808 nm laser for 8 minutes, with the light doses set at 0, 0.3, 0.5, and 1 W/cm². The cells were digested with trypsin into single cells, centrifuged to remove the supernatant, stained with PI for 30 minutes, washed with PBS, resuspended, and centrifuged to remove the supernatant. The washing steps were repeated several times, and then the cells were analyzed by flow cytometry.

The results showed that as the light dose increased, the cytotoxicity of Cyanine@DIII-PLZ4 on the cells also increased (as shown in Figure 9B).

Further, Cyanine, Cyanine@DIII-PLZ4, each at a concentration of 8.5 µM diluted in serum-free medium, and PBS as a control were used to incubate MB-49 cells for 4 h. Thereafter, the cells were treated in the same manner as described above and then irradiated with an 808 nm laser at 0.3 W/cm² for 8 min. The cells were subsequently treated with a Calcein AM/PI live/dead cell double-staining kit. Confocal microscopic observation showed that Cyanine@DIII-PLZ4 exhibited a significant cell-killing effect under laser irradiation (Fig. 9C).

### Example 7: Photodynamically Mediated Reactive Oxygen Species Generation by Cyanine@DIII-cPLZ4 in Bladder Tumor Cells

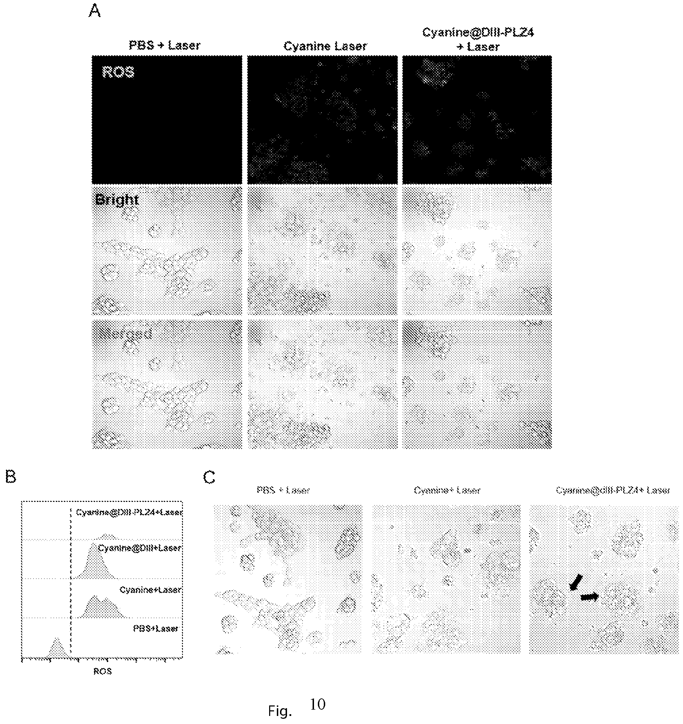

MB-49 cells were seeded in confocal culture dishes at a density of 3×10⁵ cells per dish. After incubation for 24 h, the cells were washed twice with PBS. Cyanine@DIII-cPLZ4, Cyanine and PBS were diluted with serum-free medium to a concentration of 8.5 µM. The cells were incubated with the respective treatments for 4 h, after which the drugs were removed by repeated washing with PBS, and the medium was replaced with serum-free medium. The culture dishes in the laser groups were irradiated with an 808 nm laser at 0.3 W/cm² for 8 min. Excess medium was removed, and the cells were incubated with 100 µL of 5 µM DCFH-DA dye diluted in serum-free medium for 15 min. The cells were then washed three times with PBS, 5 min each time, replenished with serum-free medium, and observed under a confocal microscope. The results showed that, compared with the control groups, the Cyanine@DIII-cPLZ4 group generated more reactive oxygen species under laser irradiation (Fig. 10A).

Flow cytometric analysis likewise demonstrated the same result. MB-49 cells were seeded in 6-well plates at a density of 2×10⁵ cells per well. The subsequent procedures for cell treatment, drug administration, and laser irradiation were the same as those described above. Observation under a conventional microscope showed that, in the Cyanine@DIII-cPLZ4 group under laser irradiation, photodynamically generated reactive oxygen species caused swelling and rupture of the tumor cell membrane (Fig. 10C). Thereafter, the adherent cells were digested with trypsin into single-cell suspensions, centrifuged at 1000 rpm for 3 min, and washed twice with PBS for 5 min each time. The cells were then incubated at room temperature for 15 min with 1 mL of 5 µM DCFH-DA dye diluted in serum-free medium, followed by centrifugation at 400 g for 3 min. After discarding the supernatant, the cells were washed twice with PBS for 5 min each time, resuspended in 1 mL of serum-free medium, and analyzed by flow cytometry. The results showed that, compared with the control groups, the Cyanine@DIII-cPLZ4 group generated more reactive oxygen species under laser irradiation (Fig. 10B).

### Example 8: Establishment of an Orthotopic Bladder Cancer Mouse Model

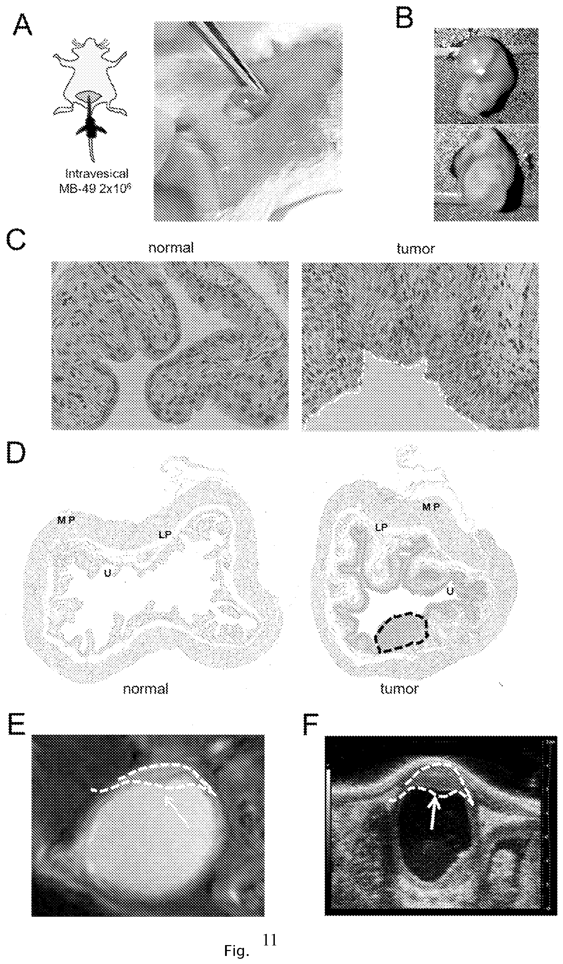

Female BALB/c mice aged 6-8 weeks were subjected to local hair removal in the abdominal region in advance, and each mouse was intraperitoneally injected with 100 µL of 7% chloral hydrate. The bladder region and surrounding skin were disinfected with alcohol and iodophor. A small incision was made at the corresponding site using surgical scissors, and the bladder was carefully exposed with sterilized forceps. An outer catheter of a 24G indwelling needle was slowly inserted through the urethra into the bladder, the urine was slowly drained, and PBS was injected into the bladder to rinse and distend the bladder. The indwelling needle was then slowly advanced through the catheter to injure the inner epithelial mucosa of the bladder (Fig. 11A). After removal of the PBS, 100 µL of MB-49 cell suspension at a concentration of 1×10⁶/mL was instilled into the bladder, and the urethra was ligated with a string for 1 h. Successful establishment of the orthotopic bladder cancer model was subsequently confirmed by multimodal analyses, including gross anatomical examination (Fig. 11B), hematoxylin-eosin (HE) staining (Figs. 11C-D), magnetic resonance imaging (Fig. 11E), and ultrasonographic imaging (Fig. 11F). The HE staining results (Figs. 11C-D) further showed urothelial cell hyperplasia without invasion into the intermediate layer or the muscular layer, indicating successful establishment of a non-muscle-invasive bladder cancer model.

### Example 9: Fluorescence Accumulation and Biodistribution of Cyanine@DIII-cPLZ4 in an Orthotopic Bladder Cancer Mouse Model

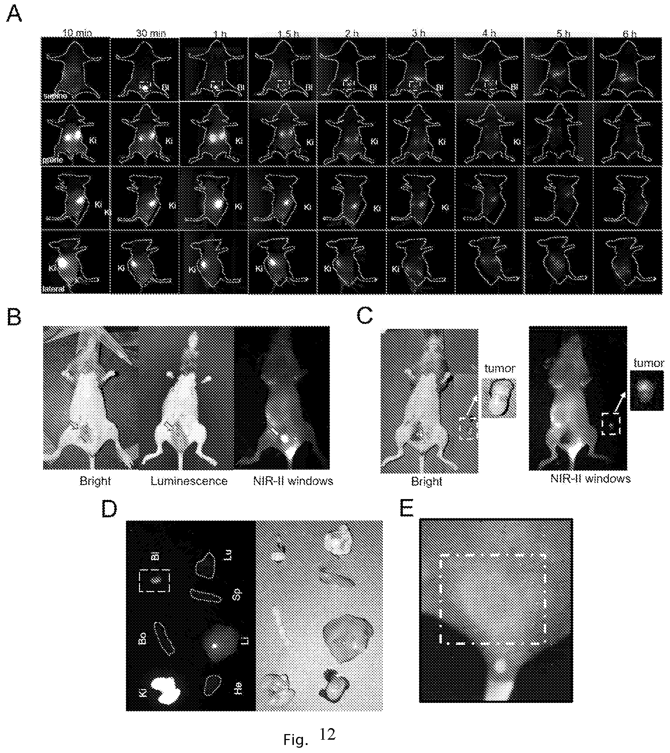

The orthotopic bladder cancer mouse model described in Example 8 was established, and the mice were subjected to whole-body hair removal. The mice were intravenously injected via the tail vein with Cyanine@DIII-PLZ4, and NIR-II fluorescence imaging was performed at 10 min, 30 min, 1 h, 2 h, 3 h, 4 h, 5 h, and 6 h after administration. As shown in Fig. 12A, the drug was metabolized to the bladder through the renal clearance pathway, and a distinct fluorescence signal was observed in the bladder tumor as early as 30 min. After repeated intravesical irrigation with PBS to remove excess dye unbound to the bladder mucosa, binding of the drug remained on the inner membrane layer of the bladder tumor, indicating effective targeting of the bladder tumor by the drug (Fig. 12E). At 3 h, the bladder tumor was excised, and retention of the fluorescence signal in the bladder was observed (Figs. 12B-D).

### Example 10: Tumoricidal Effect of Cyanine@DIII-cPLZ4 in C57BL/6 Orthotopic Bladder Cancer Mice

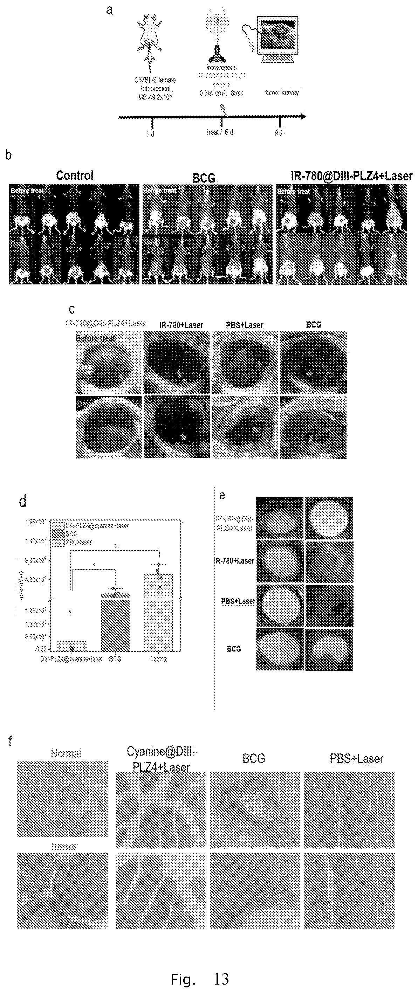

As shown in Fig. 13A, on Day 1, normal female C57BL/6 mice aged 7-8 weeks were subjected to mechanical injury of the bladder, followed by intravesical instillation of 100 µL of Luc-MB-49 murine bladder cancer cells at a concentration of 2×10⁶, thereby establishing an orthotopic bladder cancer mouse model. On Day 6, mice that had undergone local hair removal were intraperitoneally injected with 100 µL of D-luciferin substrate at 3 mg/mL, and bioluminescence imaging was performed 5 min later. Mice exhibiting bioluminescence signals were selected for grouped treatment (Fig. 13B). On the same day, MRI and ultrasonographic imaging were performed to further confirm the presence of tumor occupancy in the bladder (Fig. 13C). The tumor-bearing mice were randomly divided into three groups (n=5 per group): a Cyanine@DIII-PLZ4+Laser group, a PBS+Laser group, and a BCG group. The mice in the first two groups were intravenously injected via the tail vein with 100 µL of 85 µM Cyanine@DIII-PLZ4 and phosphate-buffered saline (PBS), respectively. After 2 h, a 24G indwelling catheter was inserted through the urethra into the bladder, and PBS was instilled through the catheter to repeatedly irrigate and remove excess dye unbound to the bladder mucosa, followed by filling the bladder with PBS to maintain bladder distension. A 400 µM optical fiber connected to an 808 nm laser was inserted into the bladder through the catheter, and under ultrasonographic guidance, the orthotopic bladder tumor was irradiated at 0.3 W/cm² for 8 min. The mice in the BCG group received intravesical instillation of 100 µL of 1 mg/mL Bacillus Calmette-Guérin diluted with sterile saline. Beginning on Day 9 (i.e. the third day after the treatment), tumor growth in the three groups was monitored by bioluminescence imaging, ultrasonography, MRI, etc. On Day 9, mice that had undergone local hair removal were intraperitoneally injected with 100 µL of D-luciferin substrate at 3 mg/mL, and bioluminescence imaging was performed 5 min later. Compared with the pre-treatment condition, no bioluminescence signal was observed in the bladder of the Cyanine@DIII-PLZ4 group, indicating tumor eradication, whereas bioluminescence signals remained in the BCG group and the PBS+Laser group, indicating that the tumors were not significantly inhibited (Fig. 13B). Quantitative bioluminescence results on Day 9 further showed that the bioluminescence signal in the Cyanine@DIII-PLZ4 group was significantly reduced as compared with the PBS+Laser group and the BCG group (Fig. 13D). On Day 12, ultrasonographic imaging and MRI showed that the intravesical tumor occupancy disappeared in the Cyanine@DIII-PLZ4 group, whereas the tumor occupancy increased in the BCG group and the PBS+Laser group (Figs. 13C and 13E). The bladders from the three groups of mice were excised, fixed in 4% paraformaldehyde, embedded in paraffin, sectioned, and subjected to HE staining. The results showed that the bladder tissue of the Cyanine@DIII-PLZ4 group exhibited no features of tumor cell proliferation, whereas tumor cell proliferation with irregular boundaries was observed in the bladder tissues of the BCG group and the PBS+Laser group (Fig. 13F).

### Example 11: Tumoricidal Effect of Cyanine@DIII-cPLZ4 in BALB/c Orthotopic Bladder Cancer Mice

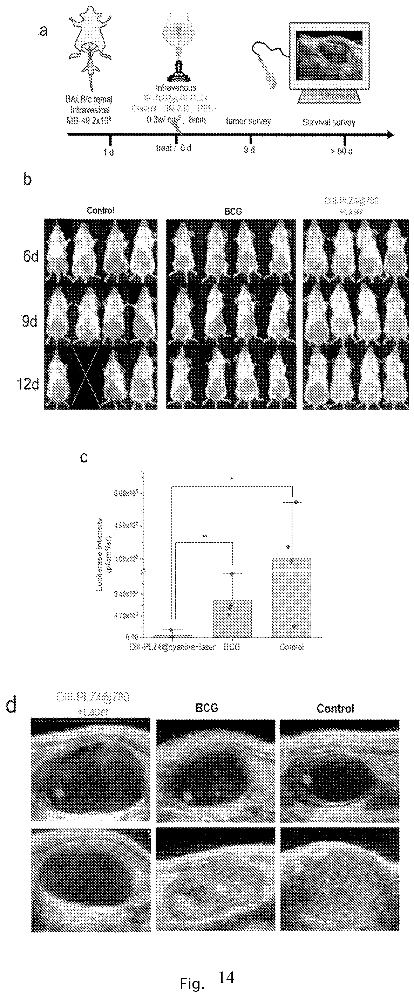

As shown in Fig. 14A, on Day 1, normal female BALB/c mice aged 7-8 weeks were subjected to mechanical injury of the bladder, followed by intravesical instillation of 100 µL of Luc-MB-49 murine bladder cancer cells at a concentration of 2×10⁶, thereby establishing an orthotopic bladder cancer mouse model. On Day 6, mice that had undergone local hair removal were intraperitoneally injected with 100 µL of D-luciferin substrate at 3 mg/mL, and bioluminescence imaging was performed 5 min later. Mice exhibiting bioluminescence signals were selected for grouped treatment (Fig. 14B). On the same day, ultrasonographic imaging was performed to further confirm the presence of tumor occupancy in the bladder (Fig. 14D). The tumor-bearing mice were randomly divided into three groups (n=5 per group): a Cyanine@DIII-PLZ4+Laser group, a PBS+Laser group, and a BCG group. The mice in the first two groups were intravenously injected via the tail vein with 100 µL of 85 µM Cyanine@DIII-PLZ4 and phosphate-buffered saline (PBS), respectively. After 2 h, a 24G indwelling catheter was inserted through the urethra into the bladder, and PBS was instilled through the catheter into the bladder to repeatedly irrigate and remove excess dye unbound to the bladder mucosa, followed by filling the bladder with PBS to maintain bladder distension. A 400 µM optical fiber connected to an 808 nm laser was inserted into the bladder through the catheter, and under ultrasonographic guidance, the orthotopic bladder tumor was irradiated at 0.3 W/cm² for 8 min. The mice in the BCG group received intravesical instillation of 100 µL of 1 mg/mL Bacillus Calmette-Guérin (BCG) diluted with sterile saline. On Days 9 and 13, mice that had undergone local hair removal were intraperitoneally injected with 100 µL of D-luciferin substrate at 3 mg/mL, and bioluminescence imaging was performed 5 min later. Compared with the pre-treatment condition, the bioluminescence signal in the bladder of the Cyanine@DIII-PLZ4 group gradually decreased until disappearance, whereas bioluminescence signals remained in the BCG group and the PBS+Laser group, and deaths occurred in these groups, indicating that the tumors were not significantly inhibited (Fig. 14B). In addition, the quantitative bioluminescence results on Day 9 (Fig. 14C) showed that, compared with the PBS+Laser group and the BCG group, the bioluminescence signal in the Cyanine@DIII-PLZ4 group was significantly reduced (P<0.05 and P<0.01, respectively). On Day 13, ultrasonographic imaging of the bladder region was performed in the three groups of mice (Fig. 14D). The images showed that the tumor had been eliminated in the Cyanine@DIII-PLZ4 group, whereas the tumor occupancy was markedly increased in the PBS+Laser group and the BCG group. In addition, on Day 60, ultrasonographic imaging and MRI of the bladder region were performed in the surviving mice of the Cyanine@DIII-PLZ4 group, and no tumor occupancy was observed, indicating that the photodynamic therapy performed in the Cyanine@DIII-PLZ4 group did not result in recurrence of bladder cancer.

### Example 12: Toxicity Evaluation of Cyanine@DIII-cPLZ4 in Healthy Mice

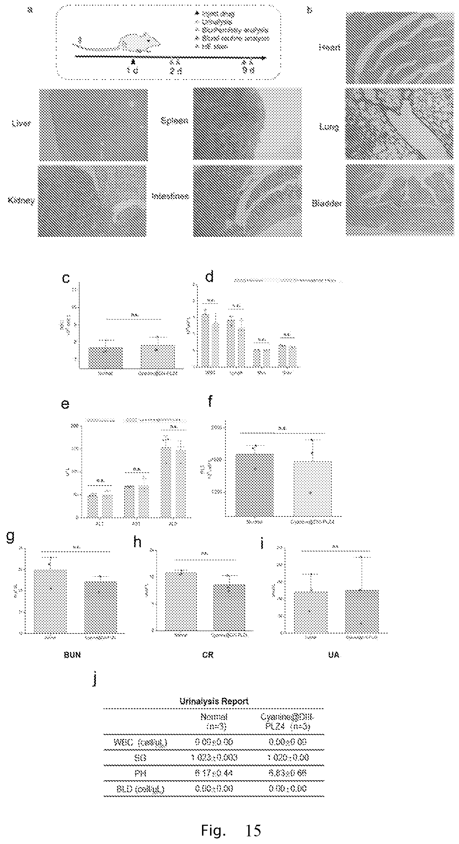

Three healthy mice were intravenously injected via the tail vein with Cyanine@DIII-PLZ4 on Day 1, and three untreated healthy mice were used as a control group. Urine, blood, and dissected organs were collected for toxicity evaluation (Fig. 15A). At 24 h after administration, a 24G indwelling catheter was inserted through the urethra into the bladder of each mouse, and urine was drained several times and collected into EP tubes, with 150-250 µL collected per tube. The samples were temporarily stored at -80°C and submitted for routine urinalysis. The results showed that indicators including WBC, SG, pH, and BLD in both the experimental group and the control group were within normal ranges (Fig. 15J). Orbital venous blood was then collected from all six mice using pre-prepared capillary tubes, and 500-700 µL of blood from each mouse was placed into a 1.5 mL centrifuge tube. The samples were allowed to stand in a 37°C incubator for 1 h, followed by centrifugation at 3000 g for 15 min at 4°C. The supernatant was collected into new centrifuge tubes, temporarily stored at -80°C, and submitted for serum biochemical analysis. The results showed that the values of ALT, AST, ALP, BUN, UA, and CR, which are indicative of liver and kidney functions, showed no significant differences between the experimental group and the control group and were all within normal ranges (Figs. 15E-I). On Day 7, orbital venous blood was collected from the six mice, with 500-700 µL of blood from each mouse placed into anticoagulant tubes, temporarily stored at 4°C, and submitted for routine blood analysis. The results showed that indicators including RBC, WBC, Lymph, Mon, and Gran in the experimental group and the control group exhibited no significant differences and were all within normal ranges (Figs. 15C-D). Thereafter, the six mice were sacrificed and dissected, and organs including the heart, liver, spleen, lung, kidney, intestine, and bladder were collected and placed in centrifuge tubes containing 4% paraformaldehyde fixative, temporarily stored at 4°C, then embedded in paraffin, sectioned, and subjected to HE staining. The results showed that administration of the drug did not produce toxic effects on the organs of normal mice (Fig. 15B).

### Example 13: Radiolabeling with Radionuclide ⁶⁴Cu and Preliminary PET Evaluation

A chelator was first prepared. p-SCN-Bn-DOTA (CAS: 127985-74-4, MedChemExpress) was purchased and dissolved in dimethyl sulfoxide (DMSO) to prepare Solution 1 at a concentration of 20 nmol/µL. All other chemicals and reagents were obtained from Sigma-Aldrich (Dorset, UK).

Proteins/polypeptides to be labeled were then prepared. The lyophilized powders of proteins DIII and DIII-PLZ4 successfully obtained according to Example 2 were dissolved in buffer (HEPES, pH 8.9, 0.1 M). A 50 µL aliquot was taken for the following reaction and designated as Solution 2.

For the labeling reaction, Solution 1 in an amount corresponding to a 40-fold molar ratio was added dropwise to Solution 2. After gentle mixing, the reaction was carried out at 37°C. The resulting mixture was incubated in a thermostatic shaker at 70 r/min for 2 h and then maintained overnight at 2-8°C to obtain Solution 3. After completion of the incubation, Solution 3 was transferred to an ultrafiltration tube and adjusted to a constant volume of 300 µL, followed by centrifugation. This operation was repeated three times, and in each step the sample was resuspended with 0.1 M ammonium acetate solution (pH 6) to remove excess p-SCN-Bn-DOTA, thereby obtaining Solution 4, with a protein concentration of about 2 mg/mL.

Preparation of ⁶⁴CuCl₂ and ⁶⁴Cu labeling was then performed. ⁶⁴Cu was produced via the ⁶⁴Ni(p,n)⁶⁴Cu reaction on a CTI RDS cyclotron (112-11 MeV). The bombarded ⁶⁴Ni target (10 mg) was dissolved from the gold-plated target using 100-150 µL of dilute hydrochloric acid. ⁶⁴Cu was isolated and purified by loading onto an anion exchange column (Biorad AG1-X8 resin) to obtain ⁶⁴CuCl2. Excess ⁶⁴Ni was eluted with 9 M HCl. Before elution of ⁶⁴Cu²⁺ with 0.1 M HCl, the pH was adjusted using 6 M HCl. The target fraction in the eluate was diluted with an equal volume of 1 M ammonium acetate solution to adjust the pH to 6. The resulting solution contained approximately 0.5 M chloride and 0.5 M acetate.

The above-prepared ⁶⁴CuCl₂ solution (37 MBq, 120 µL) was added to the above-prepared conjugation solution (Solution 4, 240 µg, 120 µL) and incubated at room temperature for 20 min.

Radiochemical yield was determined by instant thin-layer chromatography and size-exclusion high-performance liquid chromatography. For instant thin-layer chromatography, ITLC-SA (Varian) was used, with 0.1 M citrate buffer (pH 5) as the mobile phase. For size-exclusion high-performance liquid chromatography, a BioSep SEC-S-2000 column (Phenomenex, Macclesfield, UK) was used, with a thermostatic, isocratic mobile phase of 0.1 M phosphate buffer containing 50 mM EDTA at pH 7, at a flow rate of 1.0 mL/min. The retention time of unbound ⁶⁴Cu impurity was about 11 min.

Radiochemical purity was analyzed by high-performance liquid chromatography (HPLC). A 3 µL aliquot of the radioactive sample was added into a 1 mL sample vial, and the radioactivity was measured. PBS was then added to dilute the sample to 1 µCi/µL, and the sample was loaded into the sample tray for radiochemical purity determination.

The labeling effect was verified using a fluorescence spectrometer or other fluorescence imaging device to detect the labeled sample, thereby confirming the labeling efficiency and fluorescence intensity.

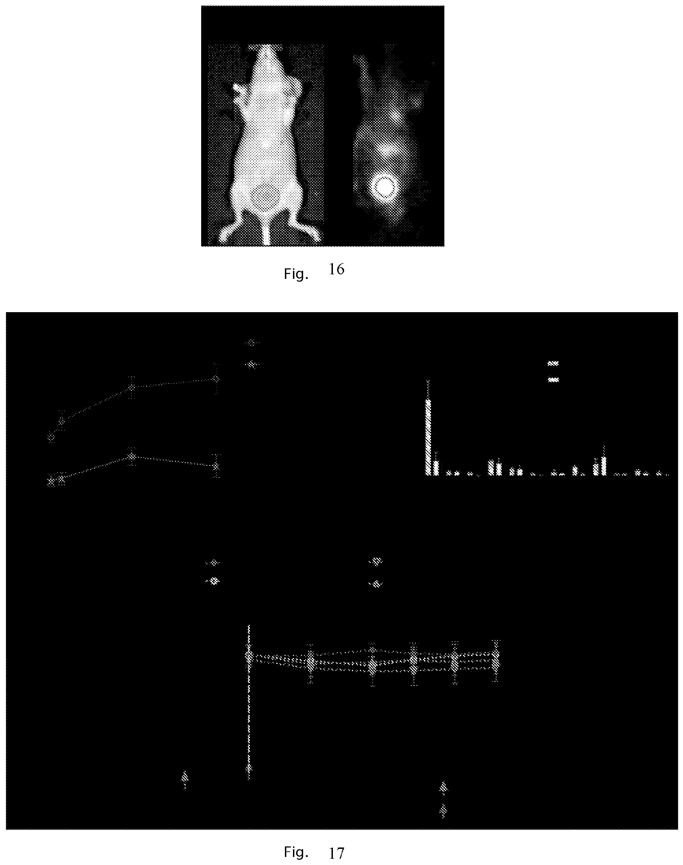

For animal experiments, a human-derived bladder cancer model was established as in Example 8 (FVB mice, 6-8 weeks). PET imaging was subsequently performed on the tumor-bearing mice. Before injection, the mice were anesthetized with isoflurane/O2 (2% v/v). The ⁶⁴Cu-labeled protein was administered by tail vein injection (4.44-5.55 MBq/120-150 µCi per mouse, 100 µL PBS). At designated time points after injection, the mice were scanned using an Inveon DPET scanner (Siemens Medical Solutions, Malvern, PA). PET images were reconstructed without attenuation correction or scatter correction. Image analysis was performed using ASI Pro VMTM software. As shown in Fig. 16, the fluorescence signal and radionuclide imaging signal in the successfully established bladder cancer model mice were completely matched, indicating that the ⁶⁴Cu-labeled DIII targeting protein was highly enriched in the tumor region.

### Example 14: Radiolabeling with Radionuclide ¹⁷⁷Lu and Preliminary Evaluation

p-SCN-Bn-DOTA (CAS: 127985-74-4, MedChemExpress) was first purchased. p-SCN-Bn-DOTA was dissolved in dimethyl sulfoxide (DMSO) to prepare Solution 1 at a concentration of 20 nmol/µL. All other chemicals and reagents were obtained from Sigma-Aldrich (Dorset, UK). Chemicals of the highest available purity, containing the lowest possible level of metal ions, were used.

Proteins/polypeptides to be labeled were then prepared. The lyophilized powders of proteins DIII and DIII-PLZ4 successfully obtained according to Example 2 were dissolved in buffer (Na₂CO₃-NaHCO₃, pH 9.5, 0.15 M). A 1.5 mL aliquot was taken for the following reaction and designated as Solution 2.

For the labeling reaction, Solution 1 in an amount corresponding to a 10-fold molar ratio was added dropwise to Solution 2. After gentle mixing, the reaction was carried out at 37°C. The reaction mixture was incubated in a thermostatic shaker at 70 r/min for 1 h. After incubation, the antibody was transferred to an ultrafiltration tube, and Buffer Solution 3 (NaOAc-Ac, pH 5.5, 0.5 M) was added to adjust the volume to 300 µL, followed by centrifugation. This operation was repeated three times to remove excess p-SCN-Bn-DOTA, thereby obtaining Solution 4.

For ¹⁷⁷Lu labeling, a [¹⁷⁷Lu]LuCl₃ solution having a radioactivity of 115.4 MBq (12 µL in volume) was added to 300 µL of the DOTA-DIII conjugate (Solution 4). The mixture was reacted in a metal bath for 45 min at 45°C. Purification was performed using an albumin affinity column according to the purification method described in Example 1.

Radiochemical yield and radiochemical purity were then determined. Radiochemical yield was measured by instant thin-layer chromatography (iTLC) as follows. A microfiber glass paper was cut into strips 10 cm in length and 1.5 cm in width, and a marking line was drawn at a position 1.5 cm from the bottom end as the sample application line. A sodium citrate-citric acid system (0.5 M, pH 5.5) was used as the developing system. After completion of the coupling reaction, the DIII variant was mixed thoroughly, and 2 µL of the radioactive sample was applied to the marking line of the microfiber glass strip. The strip was then placed into the sodium citrate developing system with the marking line facing downward, while ensuring that the marking line remained above the liquid level. The migration distance of the developer was observed under flashlight illumination. When the solvent front had migrated to a position 1 cm from the upper end, the strip was removed and allowed to dry naturally, and the radiochemical yield was then measured using Radio-iTLC.

Radiochemical purity was determined by high-performance liquid chromatography (HPLC). A 3 µL aliquot of the radioactive sample was added into a 1 mL sample vial, and the radioactivity was measured. PBS was then added to dilute the sample to 1 µCi/µL, and the sample was loaded into the sample tray for radiochemical purity determination.

For animal experiments, a bladder cancer model was established as in Example 13, followed by injection and monitoring mice. When the tumor reached an appropriate size, approximately 7-10 days after inoculation, radionuclide imaging was performed. Each mouse was injected with ¹⁷⁷Lu-DIII-tar pep at 1 µCi/µL in 200 µL PBS (pH 7.4). At different time points, the mice were anesthetized and sacrificed, and organs, including the bladder tumor, were collected for radionuclide biodistribution measurement. As shown in Fig. 17, the ¹⁷⁷Lu-labeled DIII targeting polypeptide (¹⁷⁷Lu-DIII-tar pep) exhibited significantly higher accumulation in the tumor than the control group ¹⁷⁷Lu-DIII. During long-term monitoring over a 19-day observation period, neither group produced a significant effect on mouse body weight. Six mice were included in each group.

### Example 15: Photodynamic Therapy Efficacy of Cyanine@DIII Fused Tumor-Targeting Peptide (DIII-tar pep) Administered via Intravenous Injection (i.v.) or Intravesical Infusion

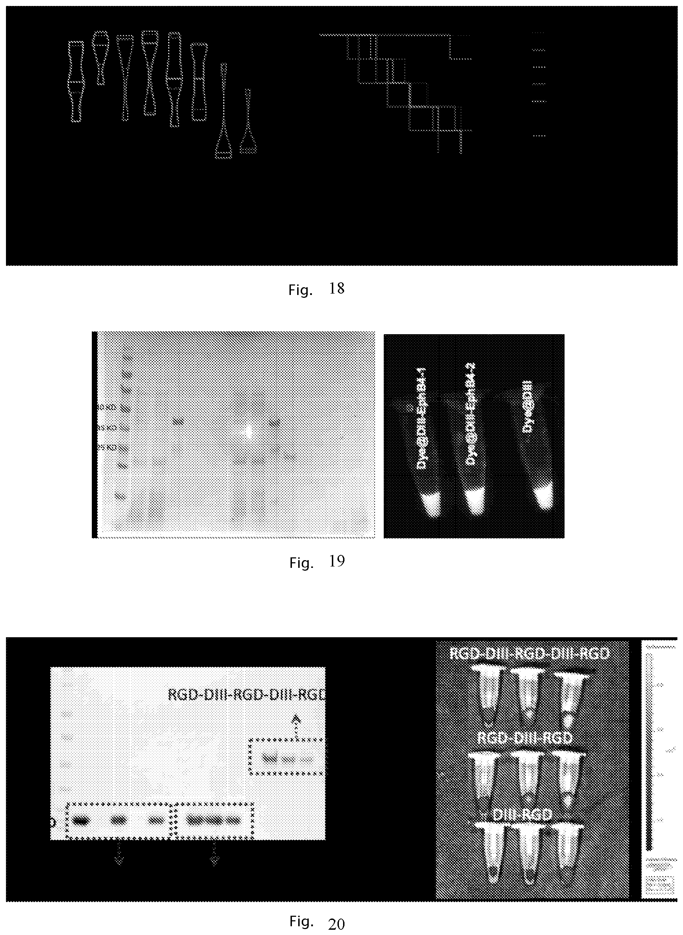

AT24 bladder cancer mouse model was established as described in Example 8. The DIII-fused tumor-targeting peptide complex (the targeting peptide used was PLZ4; labeled as DIII-tar pep in Fig. 18) developed in the present invention was incubated with a cyanine dye (the dye used was IR-783) to form a complex as described in Example 1. The complex was administered via tail vein injection or ultrasound-guided intravesical infusion. Two hours after administration, an optical fiber was inserted into the bladder under ultrasound guidance, and the tumor site was irradiated with an 808 nm laser at 0.3 W/cm² for 8 minutes. Tumor growth was monitored using a small animal imaging system, ultrasound imaging, and MRI imaging. At the experimental endpoint, tumors were excised and photographed. As shown in Fig. 18, the DIII-fused tumor-targeting peptide complex (labeled as DIII-tar pep in Fig. 18) developed in the present invention significantly inhibited tumor growth and prolonged the survival of mice, whether administered via intravenous injection (i.v.) or intravesical infusion.

The treatment groups were as follows: G1: PBS control group; G2: BCG (intravesical infusion) control group; G3: Cyanine@DIII group; G4: Cyanine@DIII-PLZ4 group; G5: PBS with laser irradiation group; G6: Cyanine@DIII with laser irradiation group; G7: Cyanine@DIII-PLZ4 (intravesical infusion) with laser irradiation group; G8: Cyanine@DIII-PLZ4 with laser irradiation group. (Unless otherwise specified as intravesical infusion, all other groups received administration via tail vein injection.)

### Example 16: Preliminary Use of DIII-EphB4-EBD Expression

DIII-EphB4-EBD was expressed, isolated, and purified using a *Pichia pastoris* expression system.

### (1). Construction of the DIII-EphB4-EBD expression vector

First, the DIII-EphB4-EBD sequence (Table 1, SEQ ID NO: 14) was cloned into the plasmid vector pPIC9K. The constructed vector plasmid was then introduced by electroporation into *Pichia pastoris* GS115 for expression. Positive transformants were screened on MD auxotrophic selection plates. The positive transformants grown on the MD plates were collected, appropriately diluted with sterile water, and plated onto YPD medium plates containing an appropriate concentration of G418 to screen high-copy-number positive transformants, thereby obtaining a DIII-EphB4-EBD expression vector.

### (2). Induced expression of DIII-EphB4-EBD

The high-copy-number DIII-EphB4-EBD-expressing strain obtained above was streaked onto an MD plate and cultured in a 30°C constant-temperature incubator for 2-3 days. After single colonies appeared, individual colonies were picked and inoculated into BMGY activation medium for activation for 12-16 h. The activated culture was then centrifuged, the culture supernatant was discarded, and the cells were transferred into BMMY expression medium for induced expression. Methanol was added daily to a final concentration of 1% for induction over 4-7 days.

### (3). Purification, Isolation, and Identification of the DIII-EphB4-EBD Protein

After induction, the supernatant of the BMMY induced expression culture was collected and sequentially filtered through 0.8 µm, 0.45 µm, and 0.22 µm membranes. The filtrate was then purified using an Albupure affinity column. The purified product was subjected to SDS-PAGE and near-infrared imaging for identification. As shown in Fig. 19 (left), the final eluate contained a highly purified product (indicated by the red dashed box). Meanwhile, as shown in Fig. 19 (right), after conjugation with the near-infrared fluorescent dye, different concentrations of DIII-EphB4-EBD (abbreviated as DIII-EphB4 in the figure), designated as Dye@DIII-EphB4-1 and Dye@DIII-EphB4-2, exhibited fluorescence characteristics consistent with those of the original DIII (Dye@DIII), indicating that the fusion strategy did not affect the fluorescence properties of DIII.

### Example 17: Preliminary Use of DIII-RGD and (DIII)m-(RGD)n Expression

### (1) Vector construction of recombinant DIII-RGD and (DIII)m-(RGD)n

The sequence information for DIII-RGD and (DIII)m-(RGD)n comprises the DIII sequence from SEQ ID NO: 1 in Table 1 combined with the RDG sequence (Arg-Gly-Asp). DIII and RDG were combined in various configurations. Here, m=2, n=3 and m=1, n=2 are used as examples for illustration. Based on the characteristics of *Escherichia coli*, the recombinant coding genes were artificially optimized, and the target genes were synthesized.

### (2) Construction of recombinant DIII-RGD and (DIII)m-(RGD)n vectors

The target gene obtained in step (1) and the pET21b(+) plasmid, which was double-digested with the restriction endonucleases HindIII and NdeI (digestion conditions: 37°C, 4 h), were subjected to agarose gel electrophoresis for identification. The target fragments were recovered using a commercial agarose gel recovery kit. The target fragments were then ligated with the pET21b(+) vector plasmid using Gibson assembly mix. The ligation products were transformed into E. coli DH5α competent cells. Positive monoclonal colonies were selected for activation and identified by colony PCR, resulting in the expression vector strains.

### (3) Induced expression of recombinant DIII-RGD and (DIII)m-(RGD)n expression vectors

Positive monoclonal colonies of the recombinant E. coli expression strain BL21(DE3) were inoculated into 5-8 mL of LB medium containing ampicillin and cultured with shaking at 37°C for 12-16 h for activation. The culture was then transferred into 500 mL of LB medium containing ampicillin and cultured at 37°C with shaking for 3-4 h until the OD600 reached 0.6-0.8. IPTG was added to a final concentration of 0.1-5 mM, and expression was induced at 18-30°C for 8-20 h. The bacterial culture was then harvested.

### (4) Isolation, purification and identification of recombinant DIII-RGD and (DIII)m-(RGD)n proteins

The bacterial culture expressing recombinant DIII-RGD and (DIII)m-(RGD)n was centrifuged at 3,000-14,000 × g for 10-20 min at 4°C. The supernatant was discarded, and the bacterial pellet was collected. Lysis buffer was added to the bacterial pellet at a ratio of 10-20 mL per 500 mL of culture. The pellet was resuspended and subjected to ultrasonication for cell lysis. After ultrasonication, the lysate was centrifuged at 10,000-15,000 × g for 10-20 min at 4°C, and the inclusion body pellet was collected. Wash buffer was added to the inclusion body pellet at a ratio of 10-20 mL per 500 mL of culture to resuspend the pellet, followed by centrifugation at 10,000-15,000 × g for 10-20 min at 4°C. This washing step was repeated 2-3 times, and the inclusion body pellet was collected. Inclusion body dissolution buffer was added to the pellet at a ratio of 5-10 mL per 500 mL of culture to dissolve the inclusion bodies. The mixture was stirred at 4°C for 12-24 h to ensure complete dissolution, followed by centrifugation at 10,000-15,000 × g for 10-20 min at 4°C. The supernatant (dissolved inclusion body solution) was collected and diluted with 50-60 volumes of protein refolding buffer, then incubated at 4°C for 1-12 h to allow refolding. The refolded target protein was purified using an Albupure affinity chromatography column to obtain the recombinant protein. Western blot analysis (Fig. 20, left) confirmed the successful expression of recombinant DIII-RGD and (DIII)m-(RGD)n. After incubation with a fluorescent dye, fluorescence intensity increased with increasing concentrations of the recombinant protein (Fig. 20, right), indicating that the expressed protein retained biological activity and exhibited fluorescence enhancement properties consistent with those of DIII.

### Example 18: Targeting Specificity of DIII-RGD and (DIII)m-(RGD)n to Three Different Tumor Cell Lines

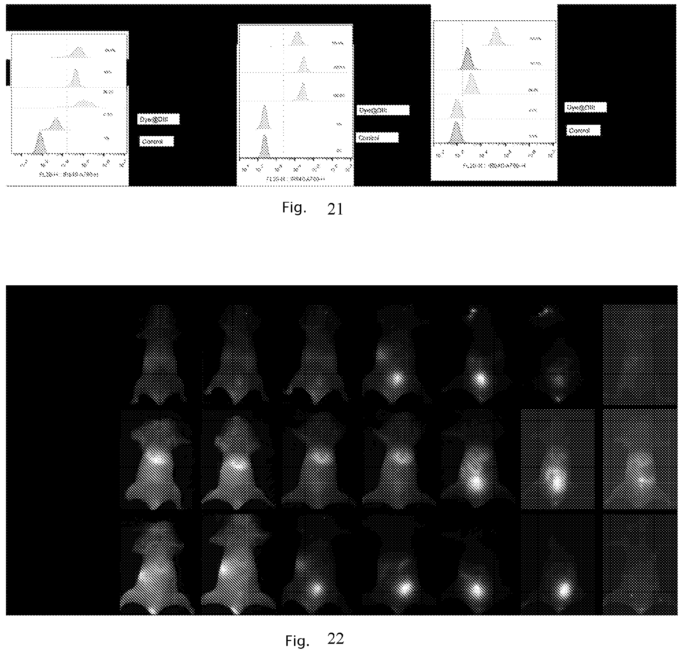

DIII-RGD, RGD-DIII-RGD, and RGD-DIII-RGD-DIII-RGD were expressed, purified, and prepared according to Example 17, and then formed into complexes with a cyanine dye (the dye used was IR-783) as described in Example 1. Flow cytometry was used to evaluate their targeting specificity to three tumor cell lines (U87MG, MDA-MB-231, and HeLa). The three tumor cell lines were separately seeded into multiple 6-well plates at a density of 2 × 10⁵ cells per well and incubated for 24 h. The culture medium was then aspirated, and the cells were gently washed 2-3 times with PBS. For each cell line, the medium was replaced with serum-free medium containing one of the following drugs: dye@DIII, dye@DIII-RGD, dye@RGD-DIII-RGD, or dye@RGD-DIII-RGD-DIII-RGD, at a concentration of 3 µM. Each group was incubated at 37°C for 6 h, with three replicate wells per group. A control group incubated with serum-free medium alone was also included. After incubation, each well was washed 3-5 times with PBS to remove unbound drugs, and the wash solution was aspirated. Adherent cells were detached by trypsinization to obtain single-cell suspensions, centrifuged at 1,000 rpm for 3 min at room temperature, resuspended in serum-free medium, and immediately analyzed by flow cytometry. The results showed that dye@DIII alone exhibited no targeting specificity to the tumor cells. In contrast, dye@DIII-RGD, dye@RGD-DIII-RGD, and dye@RGD-DIII-RGD-DIII-RGD demonstrated strong targeting ability toward all three tumor cell lines compared with both the control and dye@DIII groups (Fig. 21).

### Example 19: In Vivo Evaluation of DIII-RGD and (DIII)m-(RGD)n

RGD-DIII-RGD-DIII-RGD, RGD-DIII-RGD, and DIII-RGD successfully expressed in Example 17 were incubated with a cyanine dye (the dye used was IR-783) to form complexes as described in Example 1. The complexes were administered via tail vein injection, and their pharmacokinetic profiles in mice were recorded at various time points using NIR-II fluorescence imaging. The results, shown in Fig. 22, demonstrated that RGD-DIII-RGD-DIII-RGD, RGD-DIII-RGD, and DIII-RGD were primarily cleared via the kidneys to the bladder, and all three complexes achieved prolonged accumulation in the bladder.

Although the specific embodiments of the present invention have been described in detail, those skilled in the art will appreciate that various modifications and variations in detail may be made based on all the teachings that have been disclosed, and such changes are within the protection scope of the present invention. The full scope of the present invention is defined by the appended claims and any equivalents thereof.

## Claims

1. A complex, comprising: 1) a protein shell, wherein the protein shell is a domain III of albumin or a variant thereof, or a DIIIa subunit of albumin or a variant thereof; 2) a small molecule binding to the protein shell; and 3) a functionalized moiety linked to the protein shell, wherein the functionalized moiety is a molecule applicable for diagnosis, prevention, or treatment of a disease involving a kidney-bladder clearance pathway.

2. The complex of claim 1, wherein the albumin is a mammalian serum albumin;
preferably, the albumin is human serum albumin.

3. The complex of claim 1 or 2, wherein the small molecule is selected from one or more of a photosensitizer (e.g., a dye molecule, such as a cyanine dye molecule), a radionuclide, and a small molecule drug (e.g., a small molecule chemotherapeutic agent).

4. The complex of any one of claims 1 to 3, wherein the dye molecule has a skeleton structure selected from any one of the following structures or a derivative thereof:
Skeleton 1 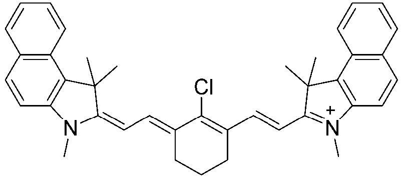
Skeleton 2 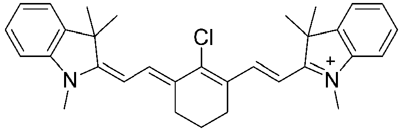
Skeleton 3 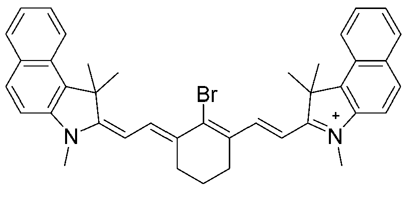
Skeleton 4 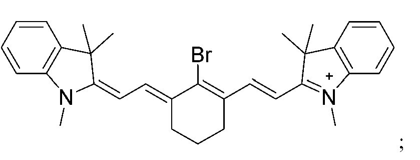
preferably, the cyanine dye molecule has a structure selected from any one of the following structures: 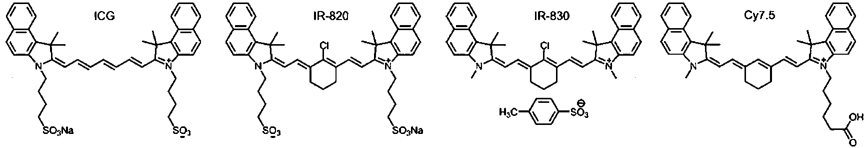 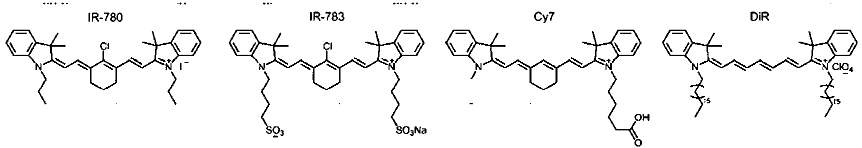 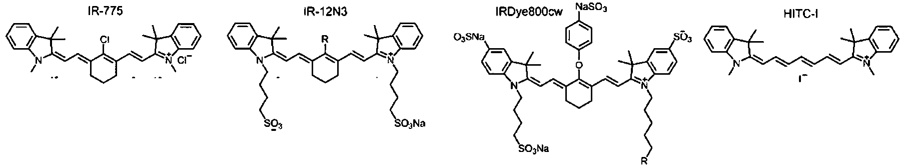 or a structure obtained by replacing Cl in the skeleton with F, Br, or I; or 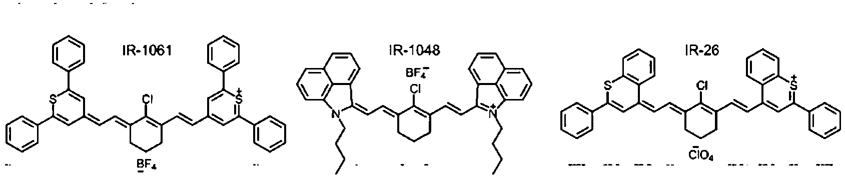 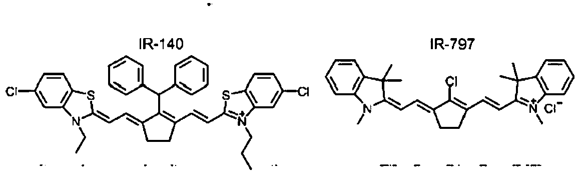 or a structure obtained by replacing Cl in the skeleton with F, Br, or I.

5. The complex of any one of claims 1 to 4, wherein the functionalized moiety is selected from:
a small molecule chemical drug applicable for diagnosis, prevention, or treatment of a disease involving a kidney-bladder clearance pathway,
a functional peptide applicable for a disease involving a kidney-bladder clearance pathway, such as a linear or cyclic peptide,
a therapeutic protein for treating a kidney or bladder disease;
preferably, the functionalized moiety is linked to the protein shell via a chemical compound, an amino acid, or a peptide;
preferably, the chemical compound is a small-molecule coupling agent for chemical conjugation (e.g., dibenzocyclooctyne-N-hydroxysuccinimidyl ester), which may also be replaced with any linker capable of effectively connecting the protein shell and the functionalized moiety;
preferably, the protein shell and the functional peptide or the therapeutic protein are prepared as a fusion protein via genetic engineering (e.g., through eukaryotic or prokaryotic expression);
preferably, the functionalized moiety is selected from short peptides targeting bladder cancer and derivatives thereof, or other small molecules, for example, a short peptide PLZ4 targeting bladder cancer, having an amino acid sequence QDGRMGF (SEQ ID NO: 10), or its cyclic peptide cQDGRMGFc (SEQ ID NO: 15), RGD or a fusion protein thereof, or prostate-specific membrane antigen (PSMA);
preferably, the functionalized moiety is a protein or another small molecule having a therapeutic effect on bladder cancer, such as the EphB4-EBD protein;
preferably, the protein shell and the functionalized moiety form a fusion/recombinant protein; more preferably, the functionalized moiety is selected from a short peptide PLZ4, a cyclic peptide cPLZ4, RGD, and an EphB4-EBD protein.

6. The complex of any one of claims 1 to 5, wherein the complex is labeled with a radionuclide (e.g., on the protein shell or the functionalized moiety);preferably, the radionuclide is an imaging radionuclide, such as ⁶⁴Cu, ¹⁸F, or ⁶⁸Ga;
preferably, the radionuclide is a therapeutic radionuclide, such as ¹⁷⁷Lu, ⁹⁰Y, ¹³¹I-, ¹²⁵I, ²²⁵Ac, or ³²P.

7. A method for preparing a functionalized protein shell comprised in the complex of any one of claims 1 to 6, comprising the following steps:
Step S1: preparing solution A comprising the domain III of albumin or a variant thereof, or the DIIIa subunit of albumin or a variant thereof according to claim 1 or 2;
Step S2: subjecting solution A to ultrafiltration centrifugation using an ultrafiltration tube, discarding filtrate, adding an alkaline solution (e.g., sodium bicarbonate solution), and vortexing at room temperature to adjust pH to an alkaline condition, thereby obtaining solution B;
Step S3: mixing a small-molecule coupling agent for chemical conjugation with solution B by vortexing at room temperature, allowing a reaction to proceed at room temperature or under heating, removing excess coupling agent by ultrafiltration centrifugation, and dissolving a product in phosphate-buffered saline (PBS) to obtain Compound 1;
Step S4: mixing Compound 1 with a raw material of the functionalized moiety (e.g., a short peptide stock solution) by vortexing at room temperature, allowing a reaction to proceed overnight at room temperature or under heating, removing excess functionalized moiety (e.g., short peptide) by ultrafiltration centrifugation, and finally dissolving a product in phosphate buffer (PBS) to obtain Compound 2.

8. A method for preparing the complex of any one of claims 1 to 7, comprising the following steps:
S1: preparing solution A comprising a functionalized protein shell, and preparing solution B comprising a small molecule (e.g., a photosensitizer (e.g., a dye molecule, such as a cyanine dye molecule), a radionuclide, or a small molecule drug (e.g., a small molecule chemotherapeutic agent)), wherein the functionalized protein shell is prepared according to the method of claim 6;
S2: mixing solution A and solution B by vortexing at room temperature, and obtaining the complex at room temperature or under heating.

9. Use of the complex of any one of claims 1 to 8 as a fluorescent probe.

10. A pharmaceutical composition comprising the complex of any one of claims 1 to 9, and optionally, comprising one or more pharmaceutically acceptable carriers or excipients;
preferably, the pharmaceutical composition is administered via intravenous injection, intravesical administration, intratumoral injection, or intraperitoneal injection;
preferably, the pharmaceutical composition is a contrast agent or a therapeutic agent.

11. A method for diagnosing, preventing, or treating a urological disease, the method comprising administering to a subject in need thereof the complex of any one of claims 1 to 6, wherein the disease includes, but is not limited to, a urological tumor or a urological injury;
preferably, the method is selected from photodynamic therapy, imaging, surgical navigation, radiotherapy, and chemotherapy.

12. Use of the complex of any one of claims 1 to 6 in preparation of a medicament for diagnosing, preventing, or treating a urological disease.

13. A kit comprising the complex of any one of claims 1 to 6.

14. A method for targeting a cell, the method comprising contacting the cell with the complex of any one of claims 1 to 6; optionally, after contacting, subjecting the cell to laser irradiation to obtain imaging of the cell.

15. Use of the complex of any one of claims 1 to 6 in preparation of a kit, wherein the kit is for targeting a cell or for obtaining imaging of a cell.

16. An imaging method, the method comprising using the complex of any one of claims 1 to 6 as an imaging agent.
